(19) Europäisches Patentamt
European Patent Office
Office européen des brevets

(11) **EP 1 017 283 B1**

(12) **EUROPEAN PATENT SPECIFICATION**

(45) Date of publication and mention
of the grant of the patent:
**15.12.2004 Bulletin 2004/51**

(51) Int Cl.7: **A01N 63/00**, A01N 65/00,
A01N 43/04, A61K 31/70,
A61K 39/00, A61K 48/00,
C12N 15/87

(21) Application number: **98906230.2**

(22) Date of filing: **13.02.1998**

(86) International application number:
**PCT/US1998/002414**

(87) International publication number:
**WO 1998/035562 (20.08.1998 Gazette 1998/33)**

(54) **POLYNUCLEOTIDE VACCINE FORMULATIONS**

POLYNUKLEOTID-IMPFSTOFF-FORMULIERUNGEN

FORMULES DE VACCINS A BASE DE POLYNUCLEOTIDES

(84) Designated Contracting States:
**AT BE CH DE DK ES FI FR GB GR IE IT LI LU NL PT SE**

(30) Priority: **14.02.1997 US 38194 P
17.03.1997 GB 9705460**

(43) Date of publication of application:
**12.07.2000 Bulletin 2000/28**

(73) Proprietor: **MERCK & CO., INC.
Rahway, New Jersey 07065 (US)**

(72) Inventors:
• **VOLKIN, David, B.**
**Rahway, NJ 07065 (US)**
• **EVANS, Robert, K.**
**Rahway, NJ 07065 (US)**
• **ULMER, Jeffrey, B.**
**Rahway, NJ 07065 (US)**
• **CAULFIELD, Michael, J.**
**Rahway, NJ 07065 (US)**

(74) Representative:
**Horgan, James Michael Frederic et al
Merck & Co., Inc.
European Patent Department
Terlings Park
Eastwick Road
Harlow, Essex CM20 2QR (GB)**

(56) References cited:
EP-A- 0 620 277          WO-A-96/00583
US-A- 5 593 972          US-A- 5 703 055
US-A- 5 703 057          US-A- 5 739 118

• ULMER J B ET AL: "TOWARD THE DEVELOPMENT OF DNA VACCINES" CURRENT OPINION IN BIOTECHNOLOGY, LONDON, GB, vol. 7, no. 6, 1996, pages 653-658, XP000872766 ISSN: 0958-1669
• BENVENISTY N ET AL: "DIRECT INTRODUCTION OF GENES INTO RATS AND EXPRESSION OF THE GENES" PROCEEDINGS OF THE NATIONAL ACADEMY OF SCIENCES OF THE UNITED STATES, vol. 83, no. 24, 1986, pages 9551-9555, XP001053329 1986 (RECD. 1987) ISSN: 0027-8424
• GUPTA R K ET AL: "ADJUVANTS FOR HUMAN VACCINES - CURRENT STATUS, PROBLEMS AND FUTURE PROSPECTS" VACCINE, BUTTERWORTH SCIENTIFIC. GUILDFORD, GB, vol. 13, no. 14, 1995, XP002913279 ISSN: 0264-410X

SEQUENCE LISTING

(1) GENERAL INFORMATION:

(i) APPLICANT: MERCK & CO., INC.

(ii) TITLE OF INVENTION: POLYNUCLEOTIDE VACCINE FORMULATIONS

(iii) NUMBER OF SEQUENCES: 14

(iv) CORRESPONDENCE ADDRESS:

    (A) ADDRESSEE: Merck & Co., Inc.
    (B) STREET: P.O. Box 2000 RY60-30
    (C) CITY: Rahway
    (D) STATE: NJ
    (E) COUNTRY: US
    (F) ZIP: 07065-0907

(v) COMPUTER READABLE FORM:

    (A) MEDIUM TYPE: Floppy disk
    (B) COMPUTER: IBM PC compatible
    (C) OPERATING SYSTEM: PC-DOS/MS-DOS
    (D) SOFTWARE: PatentIn Release #1.0, Version #1.30

(vi) CURRENT APPLICATION DATA:

    (A) APPLICATION NUMBER:
    (B) FILING DATE:
    (C) CLASSIFICATION:

(viii) ATTORNEY/AGENT INFORMATION:

    (A) NAME: Hand, J. Mark
    (B) REGISTRATION NUMBER: 36,545
    (C) REFERENCE/DOCKET NUMBER: 19907Y

(ix) TELECOMMUNICATION INFORMATION:

    (A) TELEPHONE: 732/594-3905
    (B) TELEFAX: 732/594-4720

(2) INFORMATION FOR SEQ ID NO:1:

    (i) SEQUENCE CHARACTERISTICS:

        (A) LENGTH: 23 base pairs
        (B) TYPE: nucleic acid
        (C) STRANDEDNESS: single
        (D) TOPOLOGY: linear

    (ii) MOLECULE TYPE: other nucleic acid

        (A) DESCRIPTION: /desc = "oligonucleotide"

    (xi) SEQUENCE DESCRIPTION: SEQ ID NO:1:

CTATATAAGC AGAGCTCGTT TAG         23

(2) INFORMATION FOR SEQ ID NO:2:

    (i) SEQUENCE CHARACTERISTICS:

        (A) LENGTH: 30 base pairs
        (B) TYPE: nucleic acid

(C) STRANDEDNESS: single
(D) TOPOLOGY: linear

(ii) MOLECULE TYPE: other nucleic acid

(A) DESCRIPTION: /desc = "oligonucleotide"

(xi) SEQUENCE DESCRIPTION: SEQ ID NO:2:

```
GTAGCAAAGA  TCTAAGGACG  GTGACTGCAG                                    30
```

(2) INFORMATION FOR SEQ ID NO:3:

(i) SEQUENCE CHARACTERISTICS:

(A) LENGTH: 39 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "oligonucleotide"

(xi) SEQUENCE DESCRIPTION: SEQ ID NO:3:

```
GTATGTGTCT  GAAAATGAGC  GTGGAGATTG  GGCTCGCAC                          39
```

(2) INFORMATION FOR SEQ ID NO:4:

(i) SEQUENCE CHARACTERISTICS:

(A) LENGTH: 39 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "oligonucleotide"

(xi) SEQUENCE DESCRIPTION: SEQ ID NO:4:

```
GTGCGAGCCC  AATCTCCACG  CTCATTTTCA  GACACATAC                          39
```

(2) INFORMATION FOR SEQ ID NO:5:

(i) SEQUENCE CHARACTERISTICS:

(A) LENGTH: 78 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "oligonucleotide"

(xi) SEQUENCE DESCRIPTION: SEQ ID NO:5:

```
GATCACCATG GATGCAATGA AGAGAGGGCT CTGCTGTGTG CTGCTGCTGT GTGGAGCAGT      60

CTTCGTTTCG CCCAGCGA                                                    78
```

(2) INFORMATION FOR SEQ ID NO:6:

    (i) SEQUENCE CHARACTERISTICS:

        (A) LENGTH: 78 base pairs
        (B) TYPE: nucleic acid
        (C) STRANDEDNESS: single
        (D) TOPOLOGY: linear

    (ii) MOLECULE TYPE: other nucleic acid

        (A) DESCRIPTION: /desc = "oligonucleotide"

    (xi) SEQUENCE DESCRIPTION: SEQ ID NO:6:

```
GATCTCGCTG GGCGAAACGA AGACTGCTCC ACACAGCAGC AGCACACAGC AGAGCCCTCT      60

CTTCATTGCA TCCATGGT                                                    78
```

(2) INFORMATION FOR SEQ ID NO:7:

    (i) SEQUENCE CHARACTERISTICS:

        (A) LENGTH: 33 base pairs
        (B) TYPE: nucleic acid
        (C) STRANDEDNESS: single
        (D) TOPOLOGY: linear

    (ii) MOLECULE TYPE: other nucleic acid

        (A) DESCRIPTION: /desc = "oligonucleotide"

    (xi) SEQUENCE DESCRIPTION: SEQ ID NO:7:

```
GGTACAAATA TTGGCTATTG GCCATTGCAT ACG                                   33
```

(2) INFORMATION FOR SEQ ID NO:8:

    (i) SEQUENCE CHARACTERISTICS:

        (A) LENGTH: 36 base pairs
        (B) TYPE: nucleic acid
        (C) STRANDEDNESS: single
        (D) TOPOLOGY: linear

    (ii) MOLECULE TYPE: other nucleic acid

(A) DESCRIPTION: /desc = "oligonucleotide"

(xi) SEQUENCE DESCRIPTION: SEQ ID NO:8:

CCACATCTCG AGGAACCGGG TCAATTCTTC AGCACC                36

(2) INFORMATION FOR SEQ ID NO:9:

    (i) SEQUENCE CHARACTERISTICS:

        (A) LENGTH: 38 base pairs
        (B) TYPE: nucleic acid
        (C) STRANDEDNESS: single
        (D) TOPOLOGY: linear

    (ii) MOLECULE TYPE: other nucleic acid

        (A) DESCRIPTION: /desc = "oligonucleotid

    (xi) SEQUENCE DESCRIPTION: SEQ ID NO:9:

GGTACAGATA TCGGAAAGCC ACGTTGTGTC TCAAAATC                38

(2) INFORMATION FOR SEQ ID NO:10:

    (i) SEQUENCE CHARACTERISTICS:

        (A) LENGTH: 36 base pairs
        (B) TYPE: nucleic acid
        (C) STRANDEDNESS: single
        (D) TOPOLOGY: linear

    (ii) MOLECULE TYPE: other nucleic acid

        (A) DESCRIPTION: /desc = "oligonucleotide"

    (xi) SEQUENCE DESCRIPTION: SEQ ID NO:10:

CACATGGATC CGTAATGCTC TGCCAGTGTT ACAACC                36

(2) INFORMATION FOR SEQ ID NO:11:

    (i) SEQUENCE CHARACTERISTICS:

        (A) LENGTH: 39 base pairs
        (B) TYPE: nucleic acid
        (C) STRANDEDNESS: single
        (D) TOPOLOGY: linear

    (ii) MOLECULE TYPE: other nucleic acid

        (A) DESCRIPTION: /desc = "oligonucleotide"

    (xi) SEQUENCE DESCRIPTION: SEQ ID NO:11:

GGTACATGAT CACGTAGAAA AGATCAAAGG ATCTTCTTG          39

(2) INFORMATION FOR SEQ ID NO:12:

    (i) SEQUENCE CHARACTERISTICS:

        (A) LENGTH: 35 base pairs
        (B) TYPE: nucleic acid
        (C) STRANDEDNESS: single
        (D) TOPOLOGY: linear

    (ii) MOLECULE TYPE: other nucleic acid

        (A) DESCRIPTION: /desc = "oligonucleotide"

    (xi) SEQUENCE DESCRIPTION: SEQ ID NO:12:

CCACATGTCG ACCCGTAAAA AGGCCGCGTT GCTGG          35

(2) INFORMATION FOR SEQ ID NO:13:

    (i) SEQUENCE CHARACTERISTICS:

        (A) LENGTH: 9 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:13:

Thr Tyr Gln Arg Thr Arg Ala Leu Val
1               5

(2) INFORMATION FOR SEQ ID NO:14:

    (i) SEQUENCE CHARACTERISTICS:

        (A) LENGTH: 12 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:14:

Ile Pro Gln Ser Leu Asp Ser Trp Trp Tyr Ser Leu
1               5               10

**Description**

STATEMENT REGARDING FEDERALLY SPONSORED RESEARCH

Not applicable.

REFERENCE TO MICROFICHE APPENDIX

Not applicable.

FIELD OF THE INVENTION

The present invention relates to novel vaccine formulations comprising nucleic acid molecules and an adjuvant which does not substantially bind the nucleic acid molecules, and their methods of use.

BACKGROUND OF THE INVENTION

A DNA vector containing a gene encoding a viral, bacterial, parasitic or tumor antigen has been shown to express that respective antigen in muscle cells and possibly other cell types subsequent to intramuscular injection. Such a naked DNA vector has come to be known as a polynucleotide vaccine (PNV) or DNA vaccine. The technique of using naked DNA as a prophylactic agent was reported in WO90/11092 (4 October 1990), in which naked polynucleotides were used to vaccinate vertebrates.

For example, both humoral and cell-mediated responses have been shown to occur when using DNA plasmid vectors encoding influenza antigens as a PNV, providing both homologous and cross-strain protection against a subsequent live virus challenge. The generation of both of these types of immune responses by a single vaccination approach offers a potential advantage over certain existing vaccination strategies. The use of PNVs to generate antibodies may result in an increased duration of the antibody response, and may express an antigen having both the exact sequence of the clinically circulating strain of virus as well as the proper post-translational modifications and conformation of the native protein (vs. recombinant protein). The generation of CTL responses by this means offers the benefits of cross-strain protection without the use of a live potentially pathogenic vector or attenuated virus. For a review, see Donnelly, et al, 1997, *Life Sciences* 60: 163-172.

To date, PNVs have been in the form of DNA plasmid vectors which consist of a bacterial plasmid with a strong viral promoter, the DNA fragment containing an open reading frame which expresses the antigen of interest, and a polyadenylation/transcription termination sequence. The DNA plasmid vector is transformed into and grown in a bacterial host (such as *E. coli*) then purified and injected into the host in an aqueous solution. This PNV is taken up by a host cell (such as a muscle cell) wherein the antigen of interest is expressed. The plasmid is constructed so as to lack a eukaryotic origin of replication to limit host cell replication and/or host genome integration of the PNV construct.

Benvenisty and Reshef (1986, *Proc. Natl. Acad. Sci.,* 83: 9551-9555) showed expression of DNA co-precipitated with calcium phosphate and introduced into mice intraperitoneally into liver and spleen cells.

Subsequent studies by Wolff, et al. (1990, *Science* 247: 1465-1468) showed that the intramuscular injection of DNA expression vectors without $CaPO_4$ (e.g., in saline) in mice resulted in the uptake of DNA by the muscle cells and expression of the protein encoded by the DNA. The plasmids were maintained episomally and did not replicate Wolff, et al., 1992, *Human Mol. Genetics* 1:363-369). Persistent expression has been observed after intramuscular injection in skeletal muscle of rats, fish and primates, and cardiac muscle of rats.

Intravenous injection of a DNA:cationic liposome complex in mice was shown by Zhu et al. (1993, *Science* 261: 209-211) to result in systemic expression of a cloned transgene.

It has been shown that a PNV may be delivered to the target cell by particle bombardment, whereby the polynucleotide is adsorbed onto gold microprojectiles and delivered directly intracellularly by high velocity bombardment. This method has been used to induce an immune response to human growth hormone (Tang, et al., 1992, *Nature* 356: 152-154), influenza HA (Eisenbraun, et al., 1993, *DNA Cell Biol:* 12: 791-797; Fynan, et al., 1993, *Proc. Natl. Acad. Sci.* 90: 11478-11482) and HIV gp120 (Eisenbraun, et al., 1993, *DNA Cell Biol:* 12: 791-797).

One major advantage purported of DNA vaccines is direct injection of the construct of interest in a saline or PBS solution without the addition of an adjuvant as seen with whole cell, acellular and subunit vaccines.

Adjuvants which have historically been used to enhance the immune response of classical whole cell, acellular and subunit vaccines include the mineral based compounds such as aluminum phosphate, aluminum hydroxide and calcium phosphate. These particular compounds are known in the art for a history of safe use as vaccine adjuvants, and are currently the only adjuvants approved for use in humans in the United States. Calcium phosphate is currently approved for use in humans in Europe. An aluminum phosphate adjuvant is actually amorphous aluminum hydroxy-

phosphate, $Al(OH)_m(PO_4)_n$ and an aluminum hydroxide adjuvant is actually an aluminum oxyhydroxide composition, $AlO(OH)$. Aluminum phosphate is commercially available as an amorphous aluminum hydroxyphosphate gel (known as Adju-Phos®). These adjuvants have different charges at neutral pH, with $AlO(OH)$ being positively charged and aluminum phosphate being negatively charged (see Gupta, et al., 1995, Ch.8 at page 231, in *Vaccine Design: The Subunit and Adjuvant Approach, Eds.* Powell and Newman, Plenum Press (New York and London). Vaccines containing $AlPO_4$ as an adjuvant are known to stimulate IL-4 and a $T_H2$-type of helper T cell response, as well as increasing levels of IgG1 and IgE antibodies (Vogel and Powell, 1995, Ch.7, in *Vaccine Design: The Subunit and Adjuvant Approach, Eds.* Powell and Newman, Plenum Press (New York and London) @ p. 142. Aluminum hydroxide is commercially available in crystalline form as aluminum oxyhydroxide (Alhydrogel®), and is also known as boehmite. Vaccines comprising $AlO(OH)$ as an adjuvant also stimulate IL-4, T-helper-2 subsets, as well as increasing levels of IgG1 and IgE antibodies (Vogel and Powell, 1995, Ch.7, in *Vaccine Design: The Subunit and Adjuvant Approach, Eds.* Powell and Newman, Plenum Press (New York and London) @ p. 146.

It is also known in the art that preparations of both amorphous aluminum hydroxyphosphate gel and aluminum oxyhydroxide used in commercial vaccines vary. Shirodkar, et al. (1990, *Pharm. Res.* 7(12): 1282-1288) investigated nine commercially available aluminum-containing adjuvants by X-ray diffraction, infrared spectroscopy, electron microscopy and energy dispersive spectrometry. These authors reiterate that the commercially available form of aluminum phosphate is an amorphous hydroxyphosphate and the aluminum hydroxide form is aluminum oxyhydroxide, or boehmite.

Effective adjuvanticity is known to be dependent on adsorption of the antigen of interest to an aluminum adjuvant. Studies suggest that electrostatic forces are paramount in effective absorption. Seeber, et al. (1991, *Vaccine* 9: 201-203) show that the importance of electrostatic forces is such that antigens with a high isoelectric point should be adsorbed to Adju-Phos® whereas antigens with a low isoelectric point may best be adsorbed to (Alhydrogel®).

Al-Shakhshir, et al. (1994, *Vaccine* 12(5): 472-474 show that protein adsorption to preformed aluminum adjuvants affects the surface charge characteristics of the adjuvant. Therefore, knowledge of both the adjuvant and protein surface properties are of importance in predicting the nature of a classical antigen-adjuvant vaccine formulation.

As noted above, calcium phosphate is another mineral salt which has been successfully used as an adjuvant to traditional protein vaccines. The use of calcium phosphate as an adjuvant is known and was first disclosed by Relyveld, et al. (1964, *Bull. WHO* 30: 321-325). The properties of a calcium phosphate adjuvant gel are controlled by the concentration of disodium hydrogen phosphate and calcium chloride utilized, as well the mixing rate (i.e., slower mixing rates resulting in a lower calcium to phosphate ratio). As with other adjuvants, binding to the antigen of interest is a prerequisite for enhanced immunogenicity.

Despite advances in the use of naked DNA vector-based vaccines, there is a distinct need for a pharmaceutical formulation which results in an enhanced immune response in a vertebrate host of interest. The present invention addresses this need by disclosing a DNA vaccine formulation comprising an adjuvant which does not substantially bind DNA and increases immunogenicity subsequent to vaccination of a vertebrate host.

SUMMARY OF THE INVENTION

The present invention relates to a novel vaccine formulation comprising nucleic acid molecules and an adjuvant provided in a biologically effective concentration so as to promote the effective induction of an immune response directed toward one or more specific antigens encoded by the nucleic acid molecule.

In particular, the present invention relates to a DNA vaccine formulation wherein the adjuvant comprises aluminium-based particles which are negatively charged in the DNA suspension. These particles possess a sufficient negative charge as to substantially retard binding to the nucleic acid molecule of interest. Such a DNA-adjuvant composition will increase the immune response and may decrease nuclease digestion of the DNA vaccine, within the vertebrate host subsequent to immunization.

A preferred embodiment of the present invention relates to a DNA vaccine formulation which comprises a non-DNA binding aluminium-based adjuvant generated from one or more forms of an aluminum phosphate-based adjuvant.

An especially preferred embodiment of the present invention relates to a DNA vaccine formulation wherein the aluminum phosphate-based adjuvant possesses a molar $PO_4/Al$ ratio of approximately 0.9, including but not limited to Adju-Phos®.

The nucleic acid vaccine of the present invention may include a deoxyribonucleic acid molecule (DNA), such as genomic DNA and complementary DNA (cDNA) as well as a ribonucleic acid molecule (RNA). The nucleic acid molecules comprising the vaccine formulations of the present invention preferably do not show substantial binding to the chosen adjuvant. Of course, the skilled artisan will be aware that within any such vaccine formulation, the possibility remains that a measurable, but not biologically determinative, amount of nucleic acid molecules used in the present invention may bind to the chosen adjuvant.

The DNA construct may be delivered to the host in the form of a recombinant viral vector (including but in no way limited to a recombinant adenovirus vector, a recombinant adeno-associated vector, recombinant retrovirus vector, a recombinant Sindbis virus vector, and a recombinant alphavirus vector, all known in the art). The DNA construct may also be delivered via a recombinant bacterial vector, such as recombinant BCG or Salmonella. Alternatively, the DNA may be associated with liposomes, such as lecithin liposomes or other liposomes known in the art, as a DNA-liposome mixture (see, for example, WO93/24640). However, a preferred vaccine formulation of the present invention comprises a non-viral DNA vector, most preferably a DNA plasmid-based vector. Standard recombinant DNA techniques for preparing and purifying DNA constructs are used to prepare the DNA polynucleotide constructs utilized in the exemplified PNV vaccine constructs disclosed throughout this specification.

Vaccine vectors for use in generating the vaccine formulations of the present invention, as well as practicing the related methods, include but are not necessarily limited to the DNA plasmid vectors V1, V1J, V1Jneo, VIJns, V1Jp, V1R and V1Jns-tPA.

The Example sections exemplify various polynucleotide vaccine constructs, such as a DNA plasmid vector expressing hemagglutinin (HA), a surface glycoprotein of influenza A, the nucleoprotein of influenza A, the HBsAg surface antigen from hepatitis B, as well as gp 120 and gag constructs from HIV. Therefore, it is evident that this specification gives excellent guidance to the skilled artisan to utilize the nucleic acid formulations of the present invention with an additional construction not expressly exemplified in the Example sections. Therefore, numerous other constructs representing different DNA constructs, modes of delivery, disease and antigen targets are envisioned for use in the vaccine formulations of the present invention. Examples of viral or bacterial challenges which may be amenable to either a prophylactic or therapeutic treatment include but are not limited to influenza, herpes simplex virus (HSV), human immunodeficiency virus (HIV), tuberculosis, human papilloma virus, hepatitis A, hepatitis B, and hepatitis C. It will also be within the scope of the present invention to provide prophylactic or, most likely, therapeutic treatment for non-infectious diseases, such as cancer, autoimmune disorders, and various allergies. Additionally, it will be within the purview of the skilled artisan to utilize the formulations of the present invention for any number of veterinary applications, including but not limited to rabies, distemper, foot and mouth disease, anthrax, bovine herpes simplex and bovine tuberculosis.

The present invention also relates to methods of generating an immune response in a vertebrate host, such as a human, by administering the DNA vaccine formulations of the present invention.

The term "polynucleotide" as used herein is a nucleic acid which contains essential regulatory elements such that upon introduction into a living, vertebrate cell, it is able to direct the cellular machinery to produce translation products encoded by the genes comprising the polynucleotide.

The term "substantially retard binding", "does not substantially bind", or similar language as used herein refers the concept that a small proportion of the nucleic acid may in fact bind adjuvant within the vaccine formulation. However, any such bound material does not affect the intended biological consequence of the vaccine formulations of the present invention. Any decrease in biological activity in response to such binding may easily be overcome by adjusting slightly upward the dosage given to the vertebrate host.

The term "promoter" as used herein refers to a recognition site on a DNA strand to which the RNA polymerase binds. The promoter forms an initiation complex with RNA polymerase to initiate and drive transcriptional activity. The complex can be modified by activating sequences termed "enhancers" or inhibiting sequences termed "silencers."

The term "leader" as used herein refers to a DNA sequence at the 5' end of a structural gene which is transcribed along with the gene. The leader usually results in the protein having an N-terminal peptide extension sometimes called a pro-sequence. For proteins destined for either secretion to the extracellular medium or a membrane, this signal sequence, which is generally hydrophobic, directs the protein into endoplasmic reticulum from which it is discharged to the appropriate destination.

The term "intron" as used herein refers to a portion or portions of a gene which does not encode a portion of the gene product. Introns from the precursor RNA are excised, wherein the resulting mRNA translates the respective protein.

The term "cassette" refers to the sequence of the present invention which contains the nucleic acid sequence which is to be expressed. The cassette is similar in concept to a cassette tape. Each cassette will have its own sequence. Thus by interchanging the cassette the vector will express a different sequence. Because of the restrictions sites at the 5' and 3' ends, the cassette can be easily inserted, removed or replaced with another cassette.

The term "3' untranslated region" or "3' UTR" refers to the sequence at the 3' end of a structural gene which is usually transcribed with the gene. This 3' UTR region usually contains the poly A sequence. Although the 3' UTR is transcribed from the DNA it is excised before translation into the protein.

The term "Non-Coding Region" or "NCR" refers to the region which is contiguous to the 3' UTR region of the structural gene. The NCR region contains a transcriptional termination signal.

The term "vector" refers to some means by which DNA fragments can be introduced into a host organism or host tissue. There are various types of vectors which include but are not limited to recombinant vectors, including DNA plasmid vectors, viral vectors such as adenovirus vectors, retrovirus vectors and adeno-associated virus vectors, as well as bacteriophage vectors and cosmid vectors.

The term "biologically effective amount" means sufficient PNV is injected to produce the adequate levels of the polypeptide. One skilled in the art recognizes that this level may vary.

The term "gene" refers to a segment of nucleic acid which encodes a discrete polypeptide.

The terms "pharmaceutical" and "vaccine" are used interchangeably to indicate compositions useful for inducing immune responses.

BRIEF DESCRIPTION OF THE FIGURES

Figure 1A and Figure 1B show the effect of aluminum phosphate on the generation of anti-HA antibody titers in mice at 4 weeks post 1 injection (Figure 1A) and 8 weeks post 1 injection (Figure 1B) at DNA HA doses of 0.5 μg and 10 μg.

Figure 2A and Figure 2B show a time course measurement of anti-HA antibody titers in mice after a single innoculation of FR-9502 HA DNA (A/Georgia/93), with ( ●) and without (■) aluminum phosphate injection at DNA HA doses of 0.5 μg (Figure 2A) and 10 μg (Figure 2B).

Figure 3A and Figure 3B show that a range of DNA doses enhance the immune response in mice, as measured by anti-HA antibody production after a single innoculation of FR-9502 HA DNA (A/Georgia/93) as measured by HI titer (Figure 3A) or ELISA titer (Figure 3B).

Figure 4 shows the enhancement of anti-NP antibody responses in mice after innoculation with NP plasmid DNA with or without aluminum phosphate at DNA doses of 5 μg and 50 μg at 6 weeks post 1 injection and 3 weeks post 2 injections.

Figure 5A (IL-2), Figure 5B (INF-γ), Figure 5C (IL-4) and Figure 5D (IL-10) show the effect of aluminum phosphate on respective cytokine secretion from antigen restimulation spleen cells of NP plasmid DNA inoculated mice (6 weeks post 1 injection and 3 weeks post 2 injection) at DNA doses of 5 mcg and 50 mcg with one, two or three injections.

Figure 6A - Figure 6D show the effect of aluminum phosphate on the cytotoxic T lymphocyte response after a single innoculation of NP plasmid DNA innoculation in mice: Figure 6A (5 μg DNA, 6 weeks post injection, flu-infected target cells); Figure 6B (5 μg DNA, 6 weeks post injection, peptide pulsed target cells); Figure 6C (50 μg DNA, 6 weeks post injection, flu-infected target cells); and, Figure 6D (50 μg DNA, 6 weeks post injection, peptide-pulsed target cells).

Figure 7 shows the effect of aluminum phosphate on the antibody response to inoculation of mice with a DNA vaccine (V1R.S) encoding hepatitis B surface antigen. A 1μg dose of Recombivax HB® was compared for immunogenicity with the V1R.S vaccine injected with or without 45 μg of aluminum phosphate (Adju-Phos®). Mice were injected at day 0 and day 42 with Recombivax HB® (●), 100 μg HBV DNA with adjuvant (♦), 100 μg HBV DNA without adjuvant (■), or 1μg of HBsAg (protein) without adjuvant (◊ ).

Figure 8 shows the effect of HBV DNA vaccine (V1R.S) dosing with and without adjuvant on HBsAg antibody production six weeks after a single injection of mice. Forty five μg of aluminum phosphate (AdjuPhos®) or aluminum hydroxyphosphate was added with 1 μg, 10 μg and 100 μg HBV DNA with and without adjuvant.

Figure 9 shows the effect of a second dose at day 42 (bleed at day 63) for the dosing effects disclosed for Figure 8.

Figure 10 shows the induction of a CTL response in response to DNA vaccination with V1R.S for a formulation with and without an aluminum phosphate adjuvant (45 μg/100 μl sample).

Figure 11 shows the effect of aluminum phosphate or calcium phosphate on the gp120 and gag antibody response after inoculation of mice with a HIV env/gag DNA plasmid construct, as measured by an ELISA assay.

Figure 12A and Figure 12B show a time course measurement of anti-DNA antibody titers in rhesus monkeys after a single inoculation with FR-9502 DNA as measured by geometric mean HI titer (Figure 12A) or ELISA (Figure 12B).

DETAILED DESCRIPTION OF THE INVENTION

The present invention relates to a novel vaccine formulation comprising nucleic acid molecules and an adjuvant provided in a biologically effective concentration so as to promote the effective induction of an immune response directed toward one or more specific antigens encoded by the nucleic acid molecule.

A particular embodiment of the present invention relates to a DNA vaccine formulation wherein the adjuvant comprises aluminium-based particles which are negatively charged in the DNA suspension. These particles possess a sufficient negative charge as to substantially retard binding to the nucleic acid molecule of interest. Such a DNA-adjuvant composition will increase the immune response and may decrease nuclease digestion of the DNA vaccine, within the vertebrate host subsequent to immunization.

A preferred embodiment of the present invention relates to a DNA vaccine formulation which comprises a non-DNA binding aluminium adjuvant generated from one or more forms of an aluminium phosphate-based adjuvant. The term "aluminum phosphate" is oftentimes used in the art to describe members of a continuous series of aluminum hydroxyphosphate compositions in which the molar $PO_4/Al$ ratio ranges from about 0.3 to about 0.9 (Hem and White, 1995, Ch. 9; in *Vaccine Design: The Subunit and Adjuvant Approach, Eds.* Powell and Newman, Plenum Press (New York and London). As noted throughout this specification, numerous conditions exist to generate the various aluminum hydroxyphosphate gels for use in the vaccine formulations of the present invention. For instance, the skilled artisan will note that Hem and White, *supra* at page 244-255 describe specific factors which will affect the surface charge of the resulting adjuvant. Hem and White state that generating an aluminum phosphate adjuvant with aluminum salts having a weak affinity for aluminum, such as aluminum chloride, will result in an adjuvant with a higher phosphate content than using an aluminum salt with a higher affinity toward aluminum, such as a sulfate anion. It will also be possible to affect the final adjuvant composition by controlling the speed of mixing, the speed and conditions for adjuvant precipitation, heating, and other physical manipulations known to the skilled artisan. In other words, numerous strategies are known and are available to generate an aluminum phosphate-based adjuvant which has a molar $PO_4/Al$ ratio such that the adjuvant will carry a net negative charge and would be expected to not substantially bind to DNA in the vaccine formulations of the present invention.

An especially advantageous aluminum phosphate adjuvant, albeit by no means a limiting one, is a substantially negatively charged aluminum phosphate based adjuvant wherein the molar $PO_4/Al$ is approximately 0.9. For example, Adju-Phos® is a commercially available form of amorphous aluminum hydroxyphosphate gel which represents an especially preferred adjuvant for use in the DNA vaccine formulations of the present invention. This preference depends on the fact that the amorphous aluminum hydroxyphosphate Adju-Phos® is comprised of negatively charged, micron-sized particles which do not substantially bind DNA in the formulations of the present invention.

The skilled artisan will be aware that the nature of the adjuvant and its ability to bind classic antigens is effected by the conditions whereby the adjuvant is initially precipitated, the precipitation conditions, pH, temperature, and ionic strength. These same type of component manipulations will be available to the skilled artisan to alter the surface charge of various aluminum phosphate-based adjuvants to create an adjuvant surface charge conducive to use in the DNA vaccine formulations of the present invention. Therefore, it will be within the purview of the skilled artisan to take an aluminum hydroxyphosphate adjuvant with, say for example, a molar $PO_4/Al$ ratio closer to 0.3, and alter the conditions of the vaccine formulation such that the manipulated adjuvant will possess a negative surface charge which substantially retards DNA binding. It is also within the boundary of the present invention to manipulate an aluminum hydroxide adjuvant (such as Alhydrogel®) by manipulating conditions including but not limited to adjuvant precipitation conditions, formulation buffer conditions, pH, temperature, and ionic strength. The goal of such an adjuvant manipulation will be to generate an adjuvant with a negatively charged surface such that adjuvant-DNA binding will be substantially prohibited. Therefore, the skilled artisan will understand after review of this specification that negatively charged adjuvants which inhibit substantial adjuvant-DNA binding may be generated by any number of procedures which are well known and readily available.

Also, the skilled artisan will be aware that non-commercial sources of aluminum phosphate-based adjuvants may be formed for use in the DNA vaccine formulations of the present invention. Such methods include but are in no way limited to mixing aluminum chloride and trisodium phosphate to generate aluminum phosphate. Once again, the skilled artisan is aware that the nature of the adjuvant and its ability to bind to classic antigens is affected by numerous variables, including but not limited to adjuvant precipitation conditions, formulation buffer conditions, pH, temperature, and ionic strength. These same type of component manipulations will be available to the skilled artisan to alter the surface charge of various non-commercial forms of aluminum hydroxyphosphate adjuvants to create an adjuvant surface charge conducive to use in the DNA vaccine formulations of the present invention. More specifically, these negatively charged adjuvants will inhibit substantial adjuvant-DNA binding and will promote the expected immune response upon vertebrate host vaccination. It will be within the purview of the artisan to determine an optimal adjuvant and DNA dose or dose range so as to maximise the adjuvant effect while a biologically active amount of free DNA remains in the formulation. The DNA vaccine formulations of the present invention will contain from about 1 to about 20,000 mcg of aluminium in an adjuvanted form such as aluminium phosphate, preferably from about 10 to about 10,000 mcg and most preferably from about 25 to about 2,500 mcg. Particular formulations may require particular amounts within these ranges, for example, about 20, 45, 90, 100, 200, 450, 750, 900, 1,500, 2,500, 3,500 mcg, 10,000 mcg, etc., or other amounts not listed here, may be used. It is noted that a majority of data reported for mice in the Example sections utilize a 100 µl injection of the DNA vaccine formulation. Therefore, a formulation comprising aluminum at 450 mcg/mL results in a 45 mcg dose of aluminum, and is referred throughout the specification as an adjuvant dose, such as 450 mcg/mL of Adju-Phos®. It should be noted that the term "mcg" is used interchangebly with "µg" throughout this specification to represent the unit of measurement, microgram.

The nucleic acid molecule of the present invention may include a deoxyribonucleic acid molecule (DNA), such as genomic DNA and complementary DNA (cDNA) as well as a ribonucleic acid molecule (RNA). The DNA of the present invention is associated, but preferably does not bind, an aluminium-based adjuvant.

The DNA construct may be delivered to the host in the form of a recombinant viral vector (including but in no way limited to a recombinant adenovirus vector, a recombinant adeno-associated vector, recombinant retrovirus vector, a recombinant Sindbis virus vector, and a recombinant alphavirus vector, all known in the art). The DNA construct may also be delivered via a recombinant bacterial vector, such as recombinant BCG or Salmonella. Alternatively, the DNA may be associated with lipids to form DNA-lipid complexes or with lipids in the form of liposomes, such as lecithin liposomes or other liposomes known in the art, to form DNA-liposome mixture (see, for example, WO93/24640.

However, a preferred vaccine formulation of the present invention comprises a non-viral DNA vector, most preferably a DNA plasmid-based vector. Standard recombinant DNA techniques for preparing and purifying DNA constructs are used to prepare the DNA polynucleotide constructs utilized in the exemplified PNV vaccine constructs disclosed throughout this specification. A gene of interest is ligated into an expression vector which has been optimized for polynucleotide vaccinations. Extraneous DNA is at least partially removed, leaving essential elements such as a transcriptional promoter, immunogenic epitopes, transcriptional terminator, bacterial origin of replication and antibiotic resistance gene.

The amount of expressible DNA to be introduced to a vaccine recipient will depend on the strength of the transcriptional and translational promoters used in the DNA construct, and on the immunogenicity of the expressed gene product. In general, an immunologically or prophylactically effective dose of about 1 µg to greater than about 5 mg, and preferably about 10 µg to 2 mg is administered directly into muscle tissue. Subcutaneous injection, intradermal introduction, impression through the skin, and other modes of administration such as intraperitoneal, intravenous, inhalation and oral delivery are also contemplated. It is also contemplated that booster vaccinations are to be provided. In this case, it is desirable for the DNA to be in a physiologically acceptable solution, such as, but not limited to, sterile saline or sterile buffered saline, taking into consideration the effect that pH, buffer conditions and ionic charge may have on the net surface charge of the mineral-based adjuvant used to formulate the DNA vaccines of the present invention.

Vaccine vectors for use in practicing the present invention include but are not necessarily limited to the DNA plasmid vectors V1, V1J, V1R, V1Jp, V1Jneo, VIJns, and V1Jns-tPA,

Vaccine vector V1 was constructed from pCMVIE-AKI-DHFR (Whang et al., 1987, *J. Virol.* 61: 1796). The AKI and DHFR genes were removed by cutting the vector with EcoRI and self-ligating. This vector does not contain intron A in the CMV promoter, so it was added as a PCR fragment that had a deleted internal SacI site [at 1855 as numbered in Chapman, et al., 1991, *Nuc. Acids Res.* 19: 3979). The template used for the PCR reactions was pCMVintA-Lux, made by ligating the HindIII and Nhel fragment from pCMV6a120 (see Chapman et al., *ibid*.), which includes hCMV-IE1 enhancer/promoter and intron A, into the HindIII and XbaI sites of pBL3 to generate pCMVIntBL. The 1881 base pair luciferase gene fragment (HindIII-SmaI Klenow filled-in) from RSV-Lux (de Wet et al., 1987, *Mol. Cell Biol.* 7: 725) was ligated into the SalI site of pCMVIntBL, which was Klenow filled-in and phosphatase treated. The primers that spanned intron A are: 5' primer: 5'-CTATATAAGCAGAGCTCGTTTAG-3' (SEQ ID NO:1); 3' primer: 5'-GTAGCAAAGATCTAAGGACGGTGACTGCAG-3' (SEQ ID NO:2). The primers used to remove the SacI site are: sense primer, 5'-GTATGTGTCTGAAAATGAGC<u>GT</u>GGAGATTGGGC TCGCAC-3' (SEQ ID NO:3) and the antisense primer, 5'-GTGCGAGCCCAATCTCC<u>AC</u>GCTCATTTTCAGACACATAC-3' (SEQ ID NO:4). The PCR fragment was cut with Sac I and Bgl II and inserted into the vector which had been cut with the same enzymes.

A V1J expression vector may be generated to remove the promoter and transcription termination elements from vector V1 in order to place them within a more defined context, create a more compact vector, and to improve plasmid purification yields. V1J is derived from vectors V1 and pUC18, a commercially available plasmid. V1 was digested with SspI and EcoRI restriction enzymes producing two fragments of DNA. The smaller of these fragments, containing the CMVintA promoter and Bovine Growth Hormone (BGH) transcription termination elements which control the expression of heterologous genes, was purified from an agarose electrophoresis gel. The ends of this DNA fragment were then "blunted" using the T4 DNA polymerase enzyme in order to facilitate its ligation to another "blunt-ended" DNA fragment. pUC18 was chosen to provide the "backbone" of the expression vector. It is known to produce high yields of plasmid, is well-characterized by sequence and function, and is of small size. The entire *lac* operon was removed from this vector by partial digestion with the HaeII restriction enzyme. The remaining plasmid was purified from an agarose electrophoresis gel, blunt-ended with the T4 DNA polymerase treated with calf intestinal alkaline phosphatase, and ligated to the CMVintA/BGH element described above. Plasmids exhibiting either of two possible orientations of the promoter elements within the pUC backbone were obtained. One of these plasmids gave much higher yields of DNA in *E. coli* and was designated V1J. This vector's structure was verified by sequence analysis of the junction regions and was subsequently demonstrated to give comparable or higher expression of heterologous genes compared with V1.

Construction of the V1Jneo expression vector requires removal of the ampr gene used for antibiotic selection of bacteria harboring V1J because ampicillin may not be desirable in large-scale fermenters. The ampr gene from the pUC backbone of V1J was removed by digestion with SspI and Eam1105I restriction enzymes. The remaining plasmid was purified by agarose gel electrophoresis, blunt-ended with T4 DNA polymerase, and then treated with calf intestinal alkaline phosphatase. The commercially available kan[r] gene, derived from transposon 903 and contained within the pUC4K plasmid, was excised using the PstI restriction enzyme, purified by agarose gel electrophoresis, and blunt-ended with T4 DNA polymerase. This fragment was ligated with the V1J backbone and plasmids with the kan[r] gene in either orientation were derived which were designated as V1Jneo #'s 1 and 3. Each of these plasmids was confirmed by restriction enzyme digestion analysis, DNA sequencing of the junction regions, and was shown to produce similar quantities of plasmid as V1J. Expression of heterologous gene products was also comparable to V1J for these V1Jneo vectors. V1Jneo#3, referred to as V1Jneo hereafter, was selected which contains the kan[r] gene in the same orientation as the amp[r] gene in V1J as the expression construct.

The expression vector VIJns was generated by adding an SfiI site to V1Jneo to facilitate integration studies. A commercially available 13 base pair SfiI linker (New England BioLabs) was added at the KpnI site within the BGH sequence of the vector. V1Jneo was linearized with KpnI, gel purified, blunted by T4 DNA polymerase, and ligated to the blunt SfiI linker. Clonal isolates were chosen by restriction mapping and verified by sequencing through the linker. The new vector was designated V1Jns. Expression of heterologous genes in V1Jns (with SfiI) was comparable to expression of the same genes in V1Jneo (with KpnI).

The DNA vaccine vector V1Jns-tPA was constructed in order to provide an heterologous leader peptide sequence to secreted and/or membrane proteins. Plasmid V1Jns was modified to include the human tissue-specific plasminogen activator (tPA) leader. Two synthetic complementary oligomers were annealed and then ligated into V1Jn which had been BglII digested. The sense and antisense oligomers were 5'-GATCACCATGGATGCAATGAAGAGAGGGCTC TGCTGTGTGCTGCTGCTGTGTGGAGCAGTCTTCGTTTCGCCCAGC GA-3' (SEQ ID NO:5); and, 5'-GATCTCGCTGGGCGAAACGAAGA CTGCTCCACACAGCAGCAGCACACAGCAGAGCCCTCTCTTCATT GCATCCATGGT-3' (SEQ ID NO:6). The Kozak sequence is underlined in the sense oligomer. These oligomers have overhanging bases compatible for ligation to BglII-cleaved sequences. After ligation the upstream BglII site is destroyed while the downstream BglII is retained for subsequent ligations. Both the junction sites as well as the entire tPA leader sequence were verified by DNA sequencing. Additionally, in order to conform with the consensus optimized vector V1Jns (=V1Jneo with an SfiI site), an SfiI restriction site was placed at the KpnI site within the BGH terminator region of V1Jn-tPA by blunting the KpnI site with T4 DNA polymerase followed by ligation with an SfiI linker (catalogue #1138, New England Biolabs). This modification was verified by restriction digestion and agarose gel electrophoresis.

Yet another DNA vaccine vector, V1R, may be utilized to practice the present invention. This DNA vaccine vector is a derivative of V1Jns. This vector is useful to obtain a minimum-sized vaccine vector without unneeded DNA sequences, which still retained the overall optimized heterologous gene expression characteristics and high plasmid yields that V1J and V1Jns afford. It was determined that (1) regions within the pUC backbone comprising the *E. coli* origin of replication could be removed without affecting plasmid yield from bacteria; (2) the 3'-region of the *kan*[r] gene following the kanamycin open reading frame could be removed if a bacterial terminator was inserted in its place; and, (3) ~300 bp from the 3'- half of the BGH terminator could be removed without affecting its regulatory function (following the original KpnI restriction enzyme site within the BGH element). V1R was constructed by using PCR to synthesize three segments of DNA from V1Jns representing the CMVintA promoter/BGH terminator, origin of replication, and kanamycin resistance elements, respectively. Restriction enzymes unique for each segment were added to each segment end using the PCR oligomers: SspI and XhoI for CMVintA/BGH; EcoRV and BamHI for the *kan*[r] gene; and, BclI and SalI for the *ori*[r]. These enzyme sites were chosen because they allow directional ligation of each of the PCR-derived DNA segments with subsequent loss of each site: EcoRV and SspI leave blunt-ended DNAs which are compatible for ligation while BamHI and BclI leave complementary overhangs as do SalI and XhoI. After obtaining these segments by PCR each segment was digested with the appropriate restriction enzymes indicated above and then ligated together in a single reaction mixture containing all three DNA segments. The 5'-end of the *ori*[r] was designed to include the T2 rho independent terminator sequence that is normally found in this region so that it could provide termination information for the kanamycin resistance gene. The ligated product was confirmed by restriction enzyme digestion (>8 enzymes) as well as by DNA sequencing of the ligation junctions. DNA plasmid yields and heterologous expression using viral genes within V1R appear similar to V1Jns. The net reduction in vector size achieved was 1346 bp (V1Jns = 4.86 kb; V1R = 3.52 kb). PCR oligomer sequences used to synthesize V1R (restriction enzyme sites are underlined and identified in brackets following sequence) are as follows: (1) 5'-GGTACAAATATTGGCTATTGGCCATT-GCATACG-3' (SEQ ID NO:7) [SspI]; (2) 5'-CCACATCTCGAGGAACCGGGTCAATTCTTCAGCACC-3' (SEQ ID NO:8) [XhoI] (for CMVintA/BGH segment); (3) 5'-GGTACAGATATCGGAAAGCCACGTTGTGTCTCAAAATC-3' (SEQ.ID NO: 9) [EcoRV]; (4) 5'-CACATGGATCCGTAATGCTCTGCCAGTGTT ACAACC-3' (SEQ ID NO:10) [BamHI], (for kanamycin resistance gene segment) (5) 5'-GGTACATGATCACGTAGAAAAGATCAAAGG ATCTTCTTG-3' (SEQ ID NO:11) [BclI]; (6) 5'-CCACATGTCGACCCG TAAAAAGGCCGCGTTGCTGG-3' (SEQ ID NO: 12): [SalI], (for *E. coli* origin of replication).

The Example sections exemplify various polynucleotide vaccine constructs, such as a DNA plasmid vector expressing hemagglutinin (HA), a surface glycoprotein of influenza A, the nucleoprotein of influenza A, the HBsAg surface antigen from hepatitis B, as well as gp 120 and gag constructs from HIV. Therefore, it is evident that this specification gives excellent guidance to the skilled artisan to utilize the nucleic acid formulations of the present invention with an additional construction not expressly exemplified in the Example sections. Therefore, it will be within the purview of the skilled artisan to grasp the teachings of this specification so as to use any variation in regard to the type of nucleic acid molecule used (such as DNA plasmid, recombinant viral vectors such as adenovirus, adeno-associated virus, retrovirus) as well as the type of viral or bacterial antigen expressed. Examples of viral or bacterial challenges which may be amenable to either a prophylactic or therapeutic treatment include but are not limited to influenza, herpes simplex virus (HSV), human immunodeficiency virus (HIV), tuberculosis, human papilloma virus, hepatitis A, hepatitis B, and hepatitis C. It will also be within the scope of the present invention to provide prophylactic or therapeutic treatment for non-infectious diseases, such as cancer, autoimmune disorders, and various allergies. This approach to vaccination will be applicable to tumors as well as infectious agents, since the CD8$^+$ CTL response is important for both pathophysiological processes (Tanaka, et al., 1988, *Annu. Rev. Immunol.* 6: 359). Therefore, eliciting an immune response against a protein crucial to the transformation process may be an effective means of cancer protection or immunotherapy. The generation of high titer antibodies against expressed proteins after injection of viral protein and human growth hormone DNA suggests that this is a facile and highly effective means of making vaccines that induce, either separately or in combination with other vectors, antibody and/or CTL responses. The DNA vaccine formulations of the present invention will also be useful for any number of veterinary applications, including but not limited to rabies, distemper, foot and mouth disease, anthrax, bovine herpes simplex and bovine tuberculosis.

An improved HSV polynucleotide vaccine formulation of the present invention will comprise a nucleic acid vector encoding an HSV antigen of interest, including but not limited to gB, gD, ΔgB (encoding the amino-terminal 707 aa of HSV-2 gB) and ΔgD, alone or in combination.

The vaccine formulations of the present invention may also be directed to the prophylactic treatment of human immunodeficiency virus-1 (HIV-1). It is well known that HIV-1 is the etiological agent of acquired human immune deficiency syndrome (AIDS) and related disorders. HIV-1 is an RNA virus of the Retroviridae family and exhibits the 5'LTR-*gag-pol-env*-LTR3' organization of all retroviruses. In addition, HIV-1 comprises a handful of genes with regulatory or unknown functions, including the tat and *rev* genes. The *env* gene encodes the viral envelope glycoprotein that is translated as a 160-kilodalton (kDa) precursor (gp160) and then cleaved by a cellular protease to yield the external 120-kDa envelope glycoprotein (gp120) and the transmembrane 41-kDa envelope glycoprotein (gp41). Gp120 and gp41 remain associated and are displayed on the viral particles and the surface of HIV-infected cells. Gp120 binds to the CD4 receptor present on the surface of helper T-lymphocytes, macrophages and other target cells. After gp120 binds to CD4, gp41 mediates the fusion event responsible for virus entry.

Infection begins when gp120 on the viral particle binds to the CD4 receptor on the surface of T4 lymphocytes or other target cells. The bound virus merges with the target cell and reverse transcribes its RNA genome into the double-stranded DNA of the cell. The viral DNA is incorporated into the genetic material in the cell's nucleus, where the viral DNA directs the production of new viral RNA, viral proteins, and new virus particles. The new particles bud from the target cell membrane and infect other cells.

Expression of HIV late genes such as *env* and *gag* is *rev*-dependent and requires that the *rev* response element (RRE) be present on the viral gene transcript. A secreted form of gp120 can be generated in the absence of *rev* by substitution of the gp120 leader peptide with a heterologous leader such as from tPA (tissue-type plasminogen activator), and preferably by a leader peptide such as is found in highly expressed mammalian proteins such as immunoglobulin leader peptides. A tPA-gp120 chimeric gene cloned into V1Jns efficiently expresses secreted gp120 in a transfected human rhabdomyosarcoma cell line. Monocistronic gp160 does not produce any protein upon transfection without the addition of a *rev* expression vector. Representative construct components include but are not limited to tPA-gp120$_{MN}$, gp160$_{IIIB}$, *gag*$_{IIIB}$: for anti-*gag* CTL, tPA-gp120$_{IIIB}$, tPA-gp140, and tPA-gp160 with structural mutations: V1, V2, and/or V3 loop deletions or substitutions.

The protective efficacy of polynucleotide HIV immunogens against subsequent viral challenge is demonstrated by immunization with the non-replicating plasmid DNA. This is advantageous since no infectious agent is involved, assembly of virus particles is not required, and determinant selection is permitted. Furthermore, because the sequence of *gag* and protease and several of the other viral gene products is conserved among various strains of HIV, protection against subsequent challenge by a virulent strain of HIV that is homologous to, as well as strains heterologous to the strain from which the cloned gene is obtained, is enabled.

The i.m. injection of a DNA expression vector encoding gp160 results in the generation of significant protective immunity against subsequent viral challenge. In particular, gp160-specific antibodies and primary CTLs are produced. Immune responses directed against conserved proteins can be effective despite the antigenic shift and drift of the variable envelope proteins. Because each of the HIV gene products exhibit some degree of conservation, and because CTL are generated in response to intracellular expression and MHC processing, it is predictable that many virus genes give rise to responses analogous to that achieved for gp160. Therefore, the DNA vaccine formulations of the present invention offers a means to induce cross-strain protective immunity without the need for self-replicating agents.

The ease of producing and purifying DNA constructs compares favorably with traditional methods of protein purification, thus facilitating the generation of combination vaccines. Accordingly, multiple constructs, for example encoding gp160, gp120, gp41, or any other HIV gene may be prepared, mixed and co-administered. Because protein expression is maintained following DNA injection, the persistence of B- and T-cell memory may be enhanced, thereby engendering long-lived humoral and cell-mediated immunity.

It is also within the realm of the present invention to include additional components to the nucleic acid-adjuvant comprising vaccine formulations of the present invention. For example, HIV DNA-adjuvant-based formulations may also comprise antigenic protein as well as additional known adjuvants, such as saponin, to further enhance the immune response within the vertebrate host. It is within the purview of the skilled artisan to add such components to the vaccine formulations of the present invention.

It is also within the scope of the present invention to use DNA formulations which comprise DNA vaccine constructs providing an immune response to *M. tuberculosis.* A preferred antigen is the Ag85A, the Ag85B, or the Ag85C antigen. Vaccine constructs include but are not limited to (1) a construct which contains the either the mature Ag85A, B or C coding region fused with tPA signal sequence; (2) a construct which contains the mature Ag85A, B, or C coding region with no signal sequence; (3) a construct which contains Ag85A, B, or C with its own signal sequence.

The vaccine formulations of the present invention are exemplified utilizing a DNA plasmid encoding HA from the A/Georgia/93 strain. However, the skilled artisan will be directed to the use of additional influenza genes which encode antigens of interest. Such genes include but in not necessarily limited to human influenza virus nucleoprotein, basic polymerase 1, nonstructural proteinl, hemagglutinin, matrix1, basic polymerase 2 of human influenza virus isolate A/PR/8/34, the nucleoprotein of human influenza virus isolate A/Beijing/353/89, the hemagglutinin gene of human influenza virus isolate A/Texas/36/91, or the hemagglutinin gene of human influenza virus isolate B/Panama/46/90.

It will also be known to the skilled artisan that the vaccine formulations of the present invention may comprise combinations of DNA plasmid constructs expressing HA from other clinical strains, including but not limited to, A/H1N1 (A/Texas/91), and B (B/Panama/90), as well as DNA constructs encoding the internal conserved influenza nucleoprotein (NP) and M1 (matrix) from both A (Beijing/89; H3N2) and B strains may be utilized in order to provide group-common protection against drifted and shifted antigens. The HA DNA will function by generating HA and resulting neutralizing antibodies against HA. This will be type-specific, with some increased breadth of protection against a drifted strain compared to the current licensed, protein-based vaccine. The NP and M1 constructs will result in the generation of CTL which will provide cross-strain protection with potentially lower viral loads and with acceleration of recovery from illness. The expected persistence of the DNA constructs (in an episomal, non-replicating, non-integrated form in the muscle cells) is expected to provide an increased duration of protection compared to the current vaccine.

The present invention relates to methods of generating an immune response in a vertebrate host, especially a human, wherein the vaccine formulations are administered to the host by any means known in the art of DNA vaccines, such as enteral and parenteral routes. These routes of delivery include but are not limited to intramusclar injection, intraperitoneal injection, intravenous injection, inhalation or intranasal delivery, oral delivery, sublingual administration, subcutaneous administration, transdermal administration, transcutaneous administration, percutaneous administration or any form of particle bombardment. The preferred methods of delivery are intramuscular injection, intranasal and oral based deliveries. An especially preferred method is intramuscular delivery. Regarding particle bombardment, use of aluminum adjuvants as outlined in this specification will improve the immune response produced by DNA delivered ballistically, on gold beads or as compacted particles. It will be well within the purview of the skilled artisan to deliver a formulation of the present invention as a simultaneous ballistic delivery of the DNA coated gold beads mixed with the aluminum or calcium adjuvant or as a subcutaneous or intramuscular injection of the adjuvant, followed by "gene gun" delivery of the DNA at or near the site of the adjuvant injection. The following examples are provided to further define the invention, without limiting the invention to the specifics of the examples.

EXAMPLE 1:

IN VITRO BINDING OF PLASMID DNA TO ALUMINUM ADJUVANTS

An experiment was designed to test the ability of various aluminum adjuvants to bind to plasmid DNA. Six different types of aluminum salts were examined, including aluminum hydroxide, aluminum hydroxyphosphate (precipitated in the presence of 3, 6, 12 or 24 mM sodium phosphate) and Adju-Phos® . The aluminum hydroxide (Alhydrogel®) and Adju-Phos® were purchased from Superfos Biosector, Denmark. The aluminum hydroxyphosphate adjuvants were prepared by precipitating aluminum potassium sulfate in 3mM, 6mM, 12mM and 24mM sodium phosphate, respectively. The results of this binding study is summarized in Table 1. FR-9502 is a V1Jp based DNA plasmid vector with the gene encoding HA (A/Georgia/93). The FR-9502 plasmid DNA binds to all of the aluminum salts, except for Adju-Phos® . These results were based on a 15 minute, 16 hour or 72 hour incubation period using either 5 or 100 mcg/mL plasmid DNA and 450 mcg/mL of aluminum adjuvant, at 2-8°C. For all the adjuvants except aluminum phosphate the binding studies were performed in saline because the presence of phosphate will change the surface charge of the adjuvant to become more like aluminum phosphate (Hem and White, 1995, Ch. 9, in *Vaccine Design: The Subunit and Adjuvant Approach, Eds.* Powell and Newman, Plenum Press (New York and London). The binding studies for aluminum phosphate were performed in PBS to allow a better comparison with the PBS control in the subsequent animal studies designed to examine the immune response. The samples were centrifuged and aliquots of the supernatant were taken and applied to a 1% agarose gel. Ethidium bromide staining of the gel following electrophoresis revealed the amount of total plasmid in solution by comparison to standards. It was also observed that there was no significant change in the supercoiled content of plasmid DNA after incubation with Adju-Phos®. Therefore, no significant binding of plasmid DNA to aluminum phosphate-based adjuvant such as Adju-Phos® was observed, even after 3 days of incubation, based on quantitation of the supercoiled DNA bands in the gel. In contrast, partial binding in 15 minutes and complete binding of the DNA after 3 days was observed for Alhydrogel®) and various aluminum hydroxyphosphate adjuvants tested.

TABLE 1

| Binding of plasmid DNA to aluminum adjuvants at 4 °C. | | | |
|---|---|---|---|
| Type of Adjuvant[a] | [DNA][b] | Incubation | Results |
| Al(OH)3 | 5 | 16 hrs | complete binding |
| 0.5Al(OH)x(PO4)y | " " | " " | " " |
| 1Al(OH)x(PO4)y | " " | " " | " " |
| 2Al(OH)x(PO4)y | " " | " " | " " |
| 4Al(OH)x(PO4)y | " " | " " | " " |
| AlPO4 | " " | " " | no binding observed |
| Al(OH)3 | 5 & 100 | 15 min | complete @ 5/partial @ 100 |
| 0.5Al(OH)x(PO4)y | " " | " " | " " |
| 1Al(OH)x(PO4)y | " " | " " | " " |
| 2Al(OH)x(PO4)y | " " | " " | " " |
| 4Al(OH)x(PO4)y | " " | " " | " " |
| AlPO4 | " " | " " | no binding observed |
| Al(OH)3 | 5 & 100 | 3 days | complete @ 5 and 100 |
| 0.5Al(OH)x(PO4)y | " " | " " | " " |
| 1Al(OH)x(PO4)y | " " | " " | " " |
| 2Al(OH)x(PO4)y | " " | " " | " " |
| 4Al(OH)x(PO4)y | " " | " " | " " |
| AlPO4 | " " | " " | no binding observed |

[a]Type of adjuvant: 0.5-4 refer to aluminum hydroxyphosphate prepared by precipitation in 3, 6, 12 or 24 mM sodium phosphate, respectively. The points of zero charge for aluminum hydroxide, aluminum hydroxyphosphate, and aluminum phosphate are estimated to be ~ 11, 7 and 5). The aluminum concentration was 450 mcg/mL.

[b]DNA concentration is expressed as mcg/mL.

Plasmid DNA at 5 and 100 mcg/mL was incubated in the presence and absence of 450 mcg/mL Adju-Phos in PBS buffer for 10 days at 2-8 °C. Aliquots of the DNA were then subjected to agarose gel electrophoresis and ethidium bromide staining. Densitometry was used to scan a negative of a photograph of the gel to determine the binding state of the DNA and the amount of supercoiled, open-circular and linear forms, by comparison to DNA standards. The results indicated that the DNA in the 5 mcg/mL DNA samples with and without aluminum phosphate was 96% supercoiled, while the DNA in the 100 mcg/mL DNA samples was 95% supercoiled. Therefore, the presence of aluminum phosphate did not alter the stability of the DNA over this period of time. The gel lanes containing DNA from the 5 mcg/mL DNA samples with and without aluminum phosphate contained 15.0 and 15.1 ng of DNA, respectively. The gel lanes containing DNA from the 100 mcg/mL DNA samples with and without aluminum phosphate contained 14.9 and 13.7 ng of DNA, respectively. Therefore, there was no apparent binding of the DNA to the aluminum phosphate over the 10 day incubation period.

EXAMPLE 2

INHIBITION OF NUCLEASES IN MOUSE AND HUMAN SERA BY ALUMINUM PHOSPHATE.

This section examines the ability of aluminum phosphate to inhibit endogenous nucleases present in mouse and human sera. Since aluminum phosphate carries a negative surface charge one may reason that nucleases may bind to aluminum phosphate and lengthen the lifetime of the DNA *in vivo*, after intramuscular injection. The results indicate that the addition of 450 mcg/mL aluminum phosphate (Adju-Phos®) to a PBS solution containing 5 mcg/mL DNA and either 10% human serum or 2.5% mouse serum resulted in a significant inhibition of nuclease digestion of DNA. The results also suggest that in 10% bovine serum, different proteins were bound to the DNA in the presence of aluminum phosphate than in the absence of aluminum phosphate (as suggested by the change in mobility in a 1% agarose gel).

Example 3, *infra*, shows the effect of aluminum phosphate (Adju-Phos®) on the immune response in mice. To this end, these nuclease inhibition experiments were repeated. The experimental conditions were the same as described in the previous paragraph, except for an evaluation of doubling the aluminum phosphate concentration to 900 mcg/mL. These data verify the previous results that aluminum phosphate inhibits nuclease activity in both human and mouse sera, and that increasing the aluminum phosphate concentration increases the degree of inhibition. It is also shown that lower nuclease activity was present in the supernatant of an aluminum phosphate (Adju-Phos®) / serum mixture. These data suggest that these nuclease proteins bind to aluminum phosphate in PBS, resulting in an inhibition of their activity, as evidenced by lower nuclease activity in the supernatant of an aluminum phosphate / serum mixture.

EXAMPLE 3

EFFECTS OF ALUMINUM SALTS ON HA DNA VACCINE POTENCY

*In vivo* potency studies in mice demonstrate that an aluminum phosphate formulation of DNA is substantially more potent (4- to 11-fold) than naked FR-9502 HA DNA in PBS, whereas HA DNA formulated with aluminum hydroxide or aluminum hydroxyphosphate resulted in lower responses than HA DNA in PBS (Table 2). This was true at 4 and 8 weeks after a single administration of the two doses of FR-9502 HA DNA tested; a limiting dose at which, based on numerous previous experiments, not all mice seroconvert (0.5 μg) and a moderate dose at which all mice seroconvert (10 μg). Importantly, this formulation appears to be both more potent at the lower dose and to have raised the ceiling on responses at the higher dose.

## TABLE 2
### Effect of Aluminum Adjuvants on HA DNA Potency in Mice

### Hemagglutination Inhibition

| Formulation | Dose (µg) | Wks | % serocon-version | GMT HI | SEM upper | SEM lower | P* | Fold Increase |
|---|---|---|---|---|---|---|---|---|
| PBS | 0.5 | 4 | 60 | 14.4 | 5.5 | 4.0 | | |
| Al(OH)3 | 0.5 | 4 | 0 | 6.3 | 0.0 | 0.0 | | |
| 1x AlHyd | 0.5 | 4 | 0 | 6.3 | 0.0 | 0.0 | | |
| AlPO4 | 0.5 | 4 | 100 | 58.3 | 32.0 | 20.0 | 0.025 | 4.0 |
| PBS | 10 | 4 | 100 | 40.6 | 11.3 | 8.8 | | |
| Al(OH)3 | 10 | 4 | 30 | 10.9 | 3.9 | 2.9 | | |
| 1x AlHyd | 10 | 4 | 0 | 6.3 | 0.0 | 0.0 | | |
| AlPO4 | 10 | 4 | 100 | 303.1 | 126.1 | 89.1 | 0.000097 | 7.5 |
| PBS | 0.5 | 8 | 80 | 66.0 | 46.8 | 27.4 | | |
| Al(OH)3 | 0.5 | 8 | 0 | 6.3 | 0.0 | 0.0 | | |
| 1x AlHyd | 0.5 | 8 | 0 | 6.3 | 0.0 | 0.0 | | |
| AlPO4 | 0.5 | 8 | 100 | 459.5 | 163.4 | 120.5 | 0.0038 | 7.0 |
| PBS | 10 | 8 | 100 | 81.2 | 27.2 | 20.4 | | |
| Al(OH)3 | 10 | 8 | 60 | 37.9 | 28.3 | 16.2 | | |
| 1x AlHyd | 10 | 8 | 0 | 6.3 | 0.0 | 0.0 | | |
| AlPO4 | 10 | 8 | 100 | 800.0 | 348.1 | 242.5 | 0.000059 | 9.9 |

### IgG ELISA

| Formulation | Dose (µg) | Wks | GMT ELISA | SEM upper | SEM lower | P* | Fold Increase |
|---|---|---|---|---|---|---|---|
| PBS | 0.5 | 8 | 12800 | 10211 | 5680 | | |
| AlPO4 | 0.5 | 8 | 144815 | 138639 | 70830 | 0.011 | 11.3 |
| PBS | 10 | 8 | 25600 | 15983 | 9839 | | |
| AlPO4 | 10 | 8 | 258031 | 136439 | 89248 | 0.0017 | 10.1 |

*two-sided t-test for independent samples

Female BALB/c mice (10/group) were inoculated with FR-9502 HA DNA (A/Georgia/93) at doses of 0.5 or 10 µg and antibody titers (HI and IgG ELISA) were determined at 4 and 8 weeks after a single administration.

Analysis of the immunoglobulin isotypes reveals that the enhancing effects of aluminum phosphate (Adju-Phos®) do not result in qualitative differences in the types of antibody produced by HA DNA (Table 3). Aluminum adjuvants tend to induce a strong Th2-type of helper T cell response against co-injected protein which is often accompanied by a predominance of IgG1 antibodies in mice.

## TABLE 3
### Immunoglobulin Isotype Analysis

| Formulation | Dose (µg) | Wks | GMT ELISA | | | | IgG2a:IgG1 |
|---|---|---|---|---|---|---|---|
| | | | IgG1 | IgG2a | IgG2b | IgG3 | |
| PBS | 0.5 | 8 | 2,786 | 9,700 | 696 | 303 | 3.48 |
| AlPO4 | 0.5 | 8 | 9,051 | 86,107 | 21,526 | 1,131 | 9.51 |
| PBS | 10 | 8 | 3,200 | 25,600 | 2,262 | 336 | 8.00 |
| AlPO4 | 10 | 8 | 29,863 | 221,244 | 34,836 | 1,600 | 7.41 |

Sera taken from mice (10/group) 8 weeks after inoculation of DNA with and without aluminum phosphate were analyzed for immunoglobulin isotypes by an ELISA.

Additional studies disclosed in Example Section 7 confirm that co-administration of aluminum phosphate with plasmid DNA encoding influenza HA enhanced the magnitude and duration of anti-HA antibodies in mice, compared to that induced by naked HA DNA alone. At 4, 8 and 17 weeks after a single inoculation, antibody titers, as measured by the functional assay hemagglutination inhibition (HI), were higher in mice vaccinated with the aluminum phosphate formulation of HA DNA. A wide range of aluminum phosphate and DNA doses are confirmed to be effective in mice, whether measured by HI or an ELISA. The enhancing effects of aluminum phosphate on a DNA construct encoding a second influenza antigen (nucleoprotein or NP) was also tested in mice and the data is also disclosed in Example Section 7. As before, antibody responses were enhanced 5- to 50-fold by formulation of DNA with aluminum phosphate. In addition, it is shown that cytotoxic T lymphocyte responses against NP in these mice were not detrimentally affected.

EXAMPLE 4

EFFECT OF ALUMINUM PHOSPHATE (Adju-Phos®) ON IN VIVO GENE EXPRESSION

An experiment to test the effect of (Adju-Phos®) on *in vivo* gene expression was conducted. A plasmid encoding secreted alkaline phosphatase (SEAP) previously shown to express in non-human primates was used. This experiment compared the level of SEAP in the serum 3 days after intramuscular injection of either 1 mcg or 10 mcg of SEAP plasmid DNA into mice, formulated in either PBS or PBS containing 450 mcg/mL aluminum phosphate. Ten mice were used in each group. The results suggest that the presence of aluminum phosphate did not have a significant effect on SEAP levels in the serum, 3 days post-injection. These results suggest that the increase in immune response obtained with aluminum phosphate may not have been the result of an overall increase in gene expression.

Example Sections 1-4 show that a DNA vaccine formulation comprising an aluminum-phosphate-based adjuvant and HA plasmid DNA (A/Georgia/93) in PBS substantially increased the humoral immune response to the expressed HA protein in mice (approximately 4- to 11-fold enhancement in antibody titer). In contrast, HA DNA formulated with aluminum hydroxide or aluminum hydroxyphosphate adjuvants shown to bind DNA inhibited the immune response to HA protein (compared to plasmid DNA alone in PBS). *In vitro* binding studies of plasmid DNA to different types of aluminum adjuvants demonstrated that plasmid DNA does not bind to the negatively charged aluminum phosphate (in PBS or in 0.9% saline). However, plasmid DNA does bind to the more positively charged aluminum hydroxide and more positively charged aluminum hydroxyphosphate adjuvants in saline. Therefore, aluminum phosphate-based adjuvants tending to posses a negative surface charge are effective non-binding adjuvants for DNA vaccine formulations.

EXAMPLE 5

IN VITRO BINDING OF PLASMID DNA TO ALUMINUM HYDROXYPHOSPHATE ADJUVANTS

Four solutions were prepared as shown below in Table 4 to determine if plasmid DNA binding to aluminum hydroxyphosphate could be prevented by the addition of phosphate buffer. Each solution contained plasmid DNA at 100 mcg/mL and aluminum hydroxyphosphate.

Table 4

| Solution | Formulation |
|----------|-------------|
| 1 | DNA in 0.9% NaCl |
| 2 | DNA in saline containing 450 mcg/mL Al |
| 3 | DNA in PBS (6 mM phosphate, 150 mM NaCl) with 450 mcg/mL Al |
| 4 | DNA in PBS (12 mM phosphate, 150 mM NaCl) with 450 mcg/mL Al |

The solutions were prepared, mixed by inversion and incubated at 4°C. After 15 minutes of incubation, the solutions were centrifuged in a microcentrifuge for 2 minutes to pellet the adjuvant. Aliquots of the supernatant were taken, diluted 20-fold with PBS and subjected to a UV absorbance scan from 400 to 220 nm. The DNA concentration in the supernatant was determined, based on the assumption that an absorbance of 1.0 at 260 nm is produced by DNA at 50 mcg/mL. The results are shown below in Table 5.

Table 5

| Solution | [DNA] in supernatant after 15 minutes |
|---|---|
| 1 | 96.7 mcg/mL |
| 2 | 11.8 mcg/mL |
| 3 | 100.0 mcg/mL |
| 4 | 99.4 mcg/mL |

The results indicate that most of the plasmid DNA bound to aluminum hydroxyphosphate within 15 minutes in 0.9% saline, but did not bind to the adjuvant in PBS. Aliquots of the supernatants were also analyzed by agarose gel electrophoresis after 15 minutes and 5 days of incubation at 4°C. The results indicated that there was no detectable DNA in the supernatant of solution 2 after 15 minutes or after 5 days of incubation. However, a comparison of the amount of DNA in the supernatants from solutions 1, 3 and 4 indicated that there was no significant binding of the DNA to the aluminum adjuvant in either 6 or 12 mM phosphate, over 5 days at 4°C. Therefore, it will be within the purview of the skilled artisan to utilize an adjuvant in a DNA vaccine formulation that may, in some formulations, substantially bind DNA. This adjuvant may be useful by including a phosphate buffer or other buffer that results in an inability to substantially bind DNA within this DNA vaccine formulation.

The ability of aluminum hydroxyphosphate to enhance the immune response generated by plasmid DNA containing the HA-Georgia Influenza gene has been examined in two experiments, formulated in both saline and PBS. The results (Table 6) indicate that aluminum hydroxyphospate did not enhance the immune response (based on geometric mean titers to the HA protein antigen) to the Influenza DNA vaccine if it was formulated in saline, but it did enhance the immune response if formulated in PBS. Agarose gel electrophoresis of the supernatants of these formulations indicated that the DNA was completely bound to the aluminum hydroxyphosphate in the saline formulation, but was not bound in the PBS formulation. These results show that the DNA must be in solution and not bound to the aluminum adjuvant in order to enhance the immune response to a DNA vaccine.

TABLE 6

| Enhancement of immune responses to an Influenza DNA vaccine in mice by aluminum adjuvants. | | | | | |
|---|---|---|---|---|---|
| Experiment | Formulation | 4 week GMT[a] | seroconverters | 8 week GMT[a] | seroconverters |
| I-79 | 10 mcg DNA in PBS | 70.7 | 10/10 | 132 | 10/10 |
| I-79 | 10 mcg DNA 45 mcg AlhydroxP in saline | 6.3 | 0/10 | 6 | 10/10 |
| I-79 | 10 mcg DNA 45 mcg AlPO4 in PBS | 459.5 | 10/10 | 1132 | 10/10 |
| I-99 | 10 mcg DNA in PBS | 25 | 8/10 | 12,800[b] | 10/10 |
| I-99 | 10 mcg DNA 45 mcg alhydroxP in PBS | 107 | 9/10 | 51,200[b] | 10/10 |
| I-99 | 10 mcg DNA 45 mcg AlPO4 in PBS | 229 | 10/10 | 33,779 | 10/10 |

[a] refers to the geometric mean titer to the HA protein antigen.

[b] 8-week GMT was determined by ELISA assay

EXAMPLE 6

EFFECT OF ALUMINUM PHOSPHATE ON POTENCY OF *INFLUENZA* DNA VACCINES

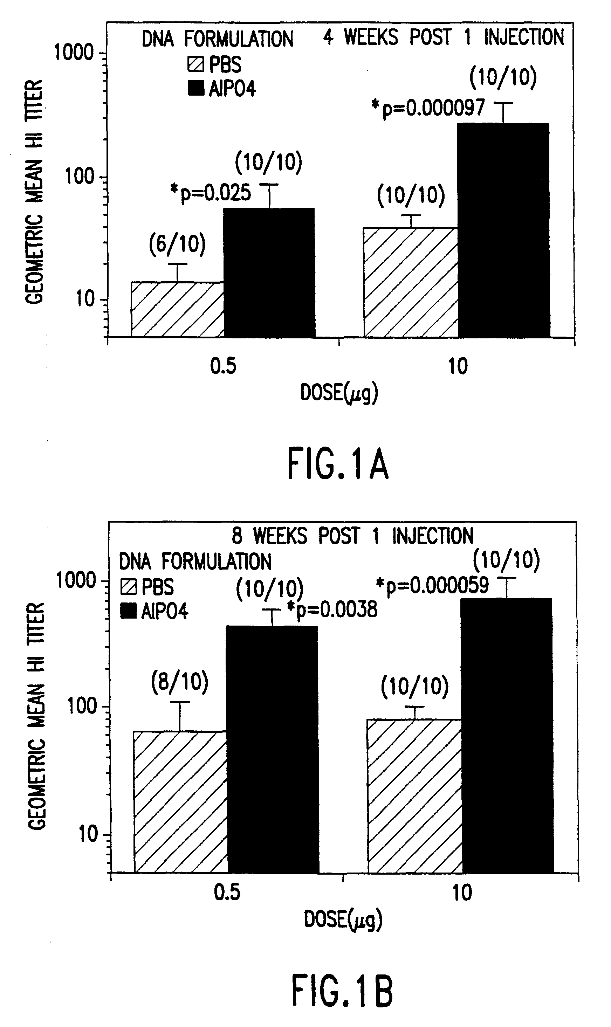

1. *Influenza HA DNA Vaccine* - Female BALB/c mice (10/group) were inoculated with FR-9502 HA DNA (A/Georgia/93) at doses of 0.5 μg or 10 μg and antibody titers (HI and IgG ELISA) were determined at 4 and 8 weeks after a single administration. Controls included inoculation with 0.5 or 10 μg of HA DNA (A/Georgia/93) in PBS. Unless indicated otherwise, $AlPO_4$ was co-administered at 450 μg/ml along with HA DNA. HA DNA potency in Figure 1A and 1B is reported as the production of neutralizing antibodies as measured *in vitro* by a hemagglutinin inhibition (HI) assay. These data show that at 4 weeks (Figure 1A) and 8 weeks (Figure 1B) post-injection, a significant enhancement of HA DNA vaccine potency is measured when utilizing a DNA vaccine formulation comprising 450 μg /ml AlPO4, with DNA at doses of both 0.5 μg and 10 μg. Table 7 shows a similar enhancement by adding an aluminum phosphate adjuvant as measured by HA ELISA.

TABLE 7

| Generation of Humoral Response in Mice | | | |
|---|---|---|---|
| DNA | Adjuvant | Dose (μg) | ELISA(GMT) |
| HA(A/Georgia/93) | PBS (None) | 0.5 | 12,800 |
| HA(A/Georgia/93) | AlPO4 | 0.5 | 144,820 |
| HA(AlGeorgia/93) | PBS (None) | 10 | 25,600 |
| HA(A/Georgia/93) | AlPO4 | 10 | 258,030 |

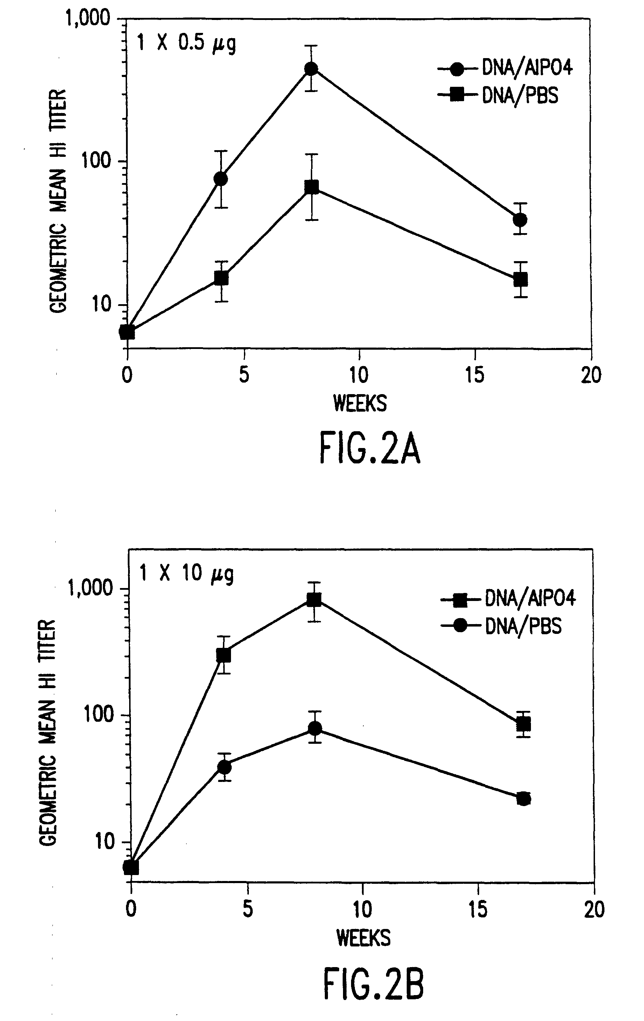

A HA DNA vaccine formulation comprising aluminum phosphate as an adjuvant did not significantly alter the IgG antibody profile. As noted *supra*, Table 3 shows that PBS- and AlPO4- based DNA vaccine formulations (measured at 0.5 and 10 μg doses at 4 and 8 weeks post-injection) result in similar isotype profiles of IgG1, IgG2a, IgG2b and IgG3 in response to HA DNA vaccination. In addition the profile of the humoral response to HA DNA vaccination, the duration of the response in mice also indicates that the rise and fall of HA neutralizing antibodies follows a similar path, regardless of whether the formulation contained PBS or AlPO4. Data in Figure 2A (0.5 μg HA DNA) and Figure 2B (10 μg HA DNA) show induction of HA neutralizing antibodies at 4, 8 and 17 weeks post-infection. In both PBS- and AlPO4- based DNA vaccine formulations, a drop in HA antibodies is seen from 8 weeks post-injection to 17 weeks post-injection.
Additional experiments show that the optimal effect of AlPO4 as an adjuvant to DNA vaccination procedures occurs when the DNA and AlPO4 are co-administered to the host. Table 8 compares the ability of HA DNA to elicit neutralizing antibodies when AlPO4 is either co-injected with the DNA or administered to mice three days prior to 3 days after DNA immunization.

TABLE 8

| Effect of Co-Administration of AlPO4/HA DNA on Enhancement of HI Titer in Mice | | |
|---|---|---|
| DNA (10mcg) | $AlPO_4$ / DNA Admin.[1] | HI Titer (GMT)[2] |
| HA(A/Georgia/93) | none (PBS) | 25 |
| HA(A/Georgia/93) | AlPO4 -co-injected | 229 |
| HA(A/Georgia/93) | AlPO4 - 3 d prior | 66 |
| HA(A/Georgia/93) | AlPO4 - 3 d after | 35 |

[1]$AlPO_4$ at 450 mcg/ml.
[2]At 4 weeks post-injection.

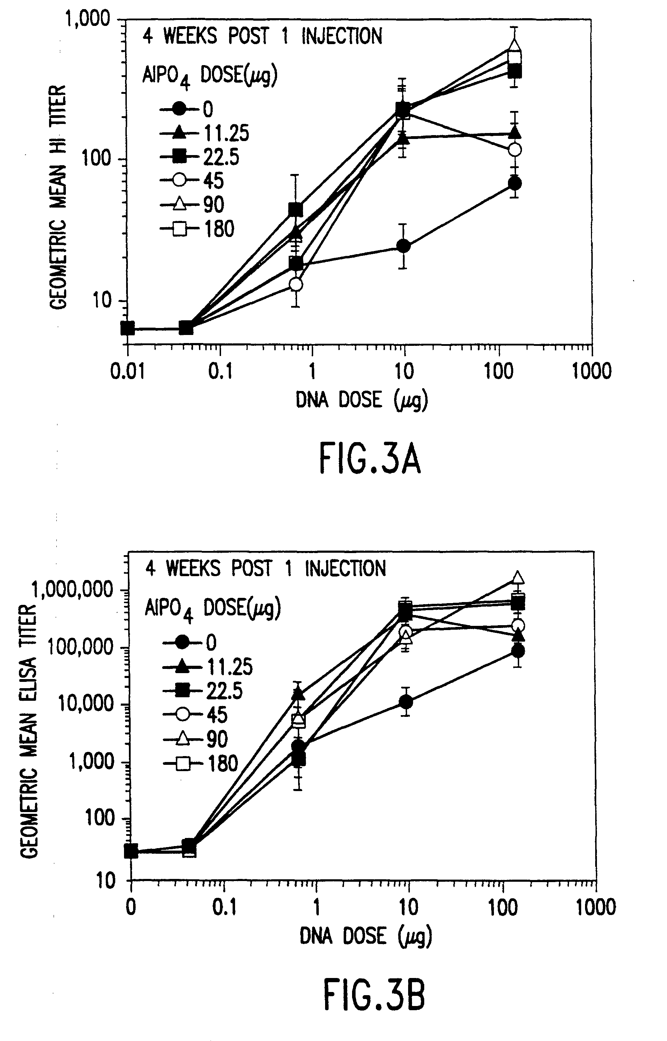

Similar results were recorded when HA antibody production was measured by an HA ELISA assay. These data show that the optimal time of administering AlPO4 as a DNA vaccine adjuvant is at or substantially near the time that the DNA vaccine is administered. Therefore, the DNA/AlPO4 formulations of the present invention provide a preferred formulation for stimulating an *in vivo* humoral response following DNA vaccination.
Additional experiments show that AlPO4 acts as an adjuvant over a wide range of concentrations which may be envisioned by the skilled artisan. Figure 3A and Figure 3B show that various AlPO4 concentrations co-administered within various dose ranges of HA DNA promote an enhanced humoral response at least 4 weeks post-injection. It is evident from these results that a wide AlPO4 dose range will be effective in providing the DNA adjuvant effect disclosed and exemplified within this specification. Therefore, the data presented in this Example Section show that AlPO4 acts as a adjuvant to significantly increase humoral responses upon DNA vaccination. This increased humoral response is not dependent upon specific dose combinations of adjuvant and DNA. Instead, higher DNA doses tend to result in somewhat more pronounced antibody production up to about a dose of 10 µg DNA in mice, whereas the adjuvant effect of AlPO4 remains steady over a large dose range. This data serves as an effective guidepost to the skilled artisan in determining DNA and adjuvant dose ranges for the host of interest, including but not limited to human and/or veterinary applications.

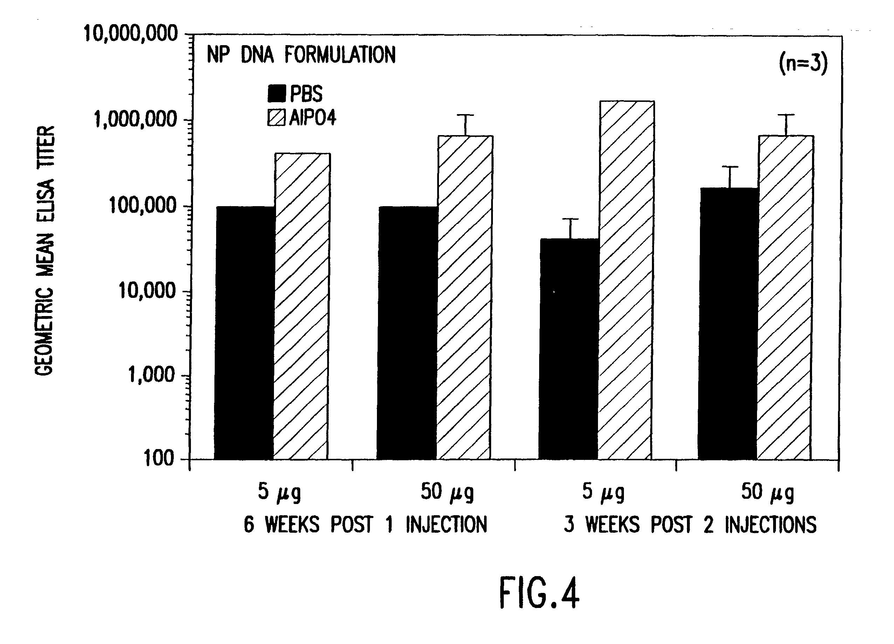

2. *Influenza NP DNA Vaccine* - Female BALB/c mice (10/group) were inoculated with a DNA plasmid encoding nucleoprotein (NP) from influenza virus A/PR/8/34 (H1N1) at doses of 0.5 µg or 50 µg and anti-NP titers were determined at 6 weeks after a single injection and at 3 weeks post two injections. Unless indicated otherwise, AlPO4 was co-administered at 450 µg /ml along with NP DNA. NP DNA potency is reported in Figure 4 as anti-NP antibodies measured as the geometric mean ELISA titer. Serum samples were collected from groups of 3 mice at the time of sacrifice for cellular immune responses. These data show that anti-NP antibody production in response to innoculation with a NP DNA plasmid construct is increased when utilizing a DNA vaccine formulation comprising 450 µg/ml AlPO4, with DNA at doses of both 0.5 µg and 50 µg.

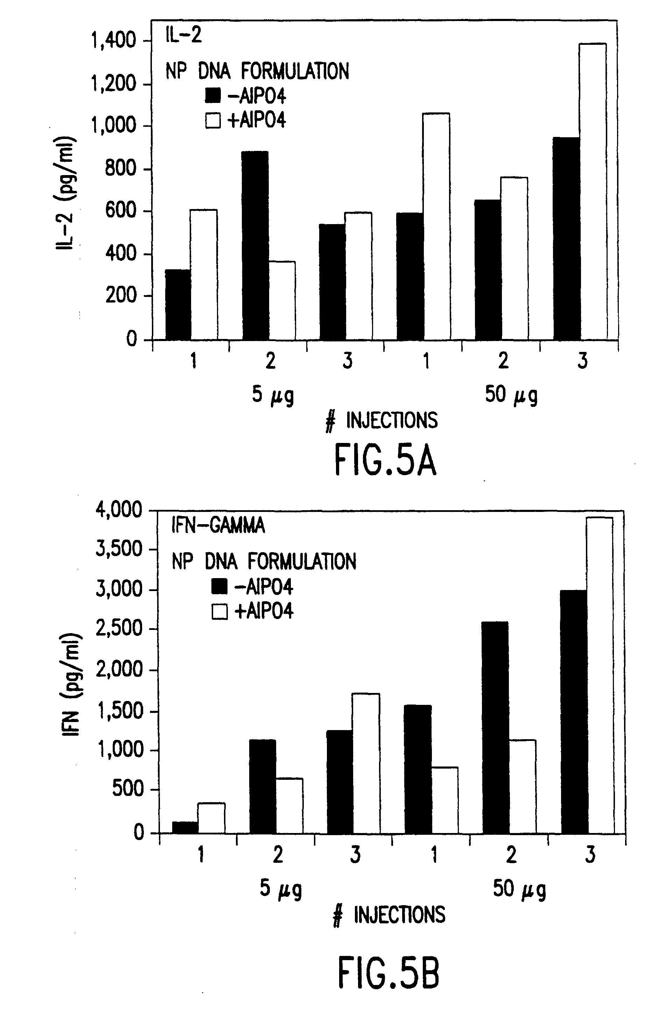

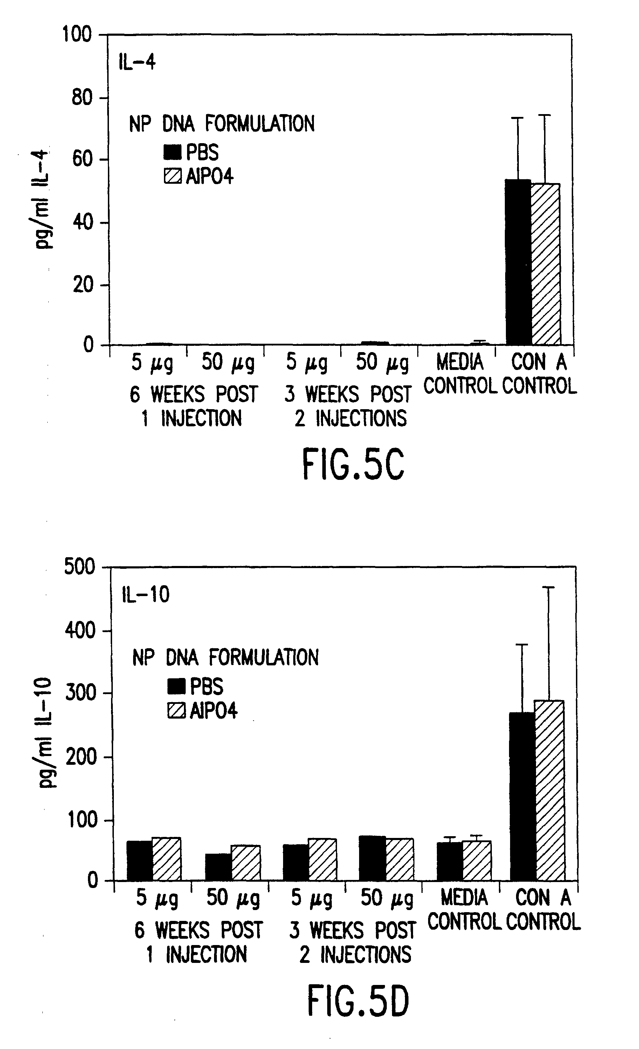

Figure 5A (IL-2), Figure 5B (INF-γ), Figure 5C (IL-4) and Figure 5D (IL-10) show that innoculation of mice with a NP DNA plasmid/AlPO4 vaccine formulation provided no significant alteration of cytokine secretion as compared to a NP DNA plasmid/PBS formulation injected at identical doses, as measured from spleen cells pooled from 3 mice/group.

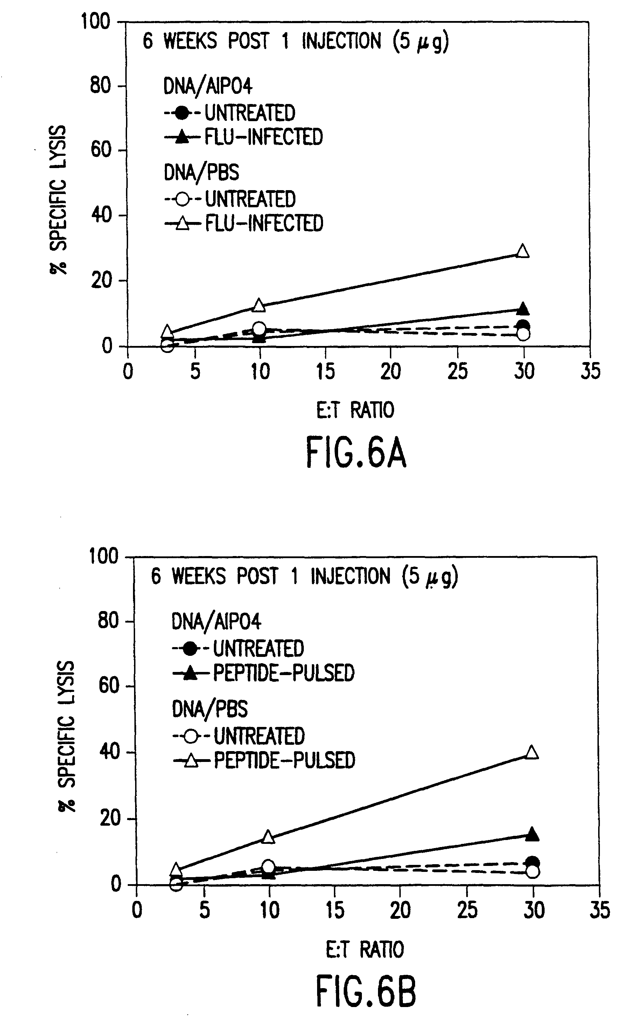

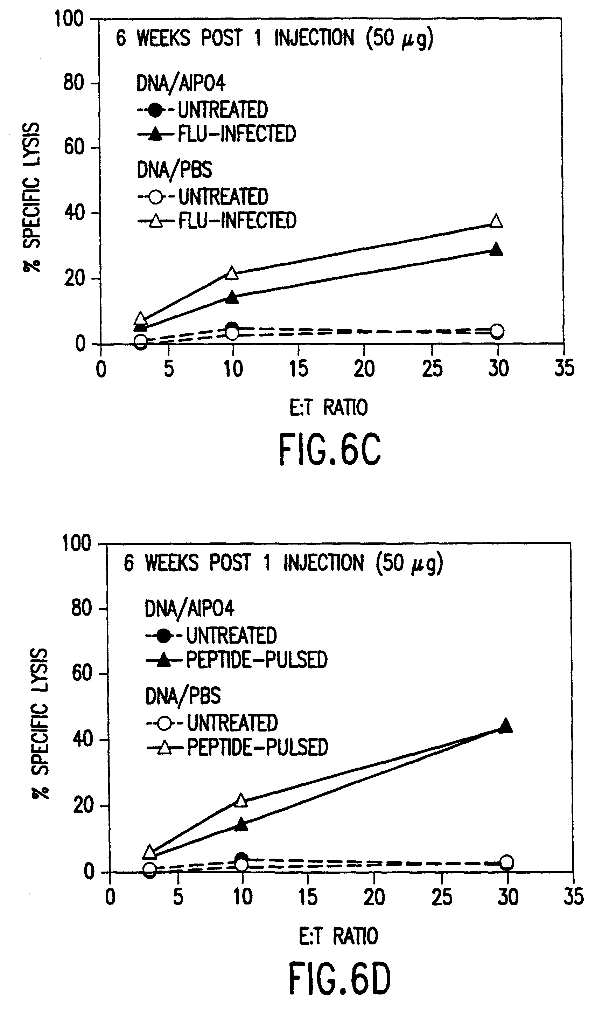

In order to show the extent of a cellular response to innoculation with a NP DNA plasmid construct, with or without the addition of AlPO4, cytotoxic T lymphocytes were generated from mice that had been immunized with DNA or that had recovered from infection with A/PR/8/34. Control cultures were derived from mice that had been injected with control DNA and from uninjected mice. Single cell suspensions were prepared from pools of 3 spleens/group, red blood cells were removed by lysis with ammonium chloride, and spleen cells were cultured in RPMI 1640 supplemented with 10% Fetal Bovine Serum (FBS), 100 U/ml penicillin, 100 µg/ml streptomycin, 0.01 M HEPES (pH 7.5), and 2 mM 1-glutamine. An equal number of autologous, irradiated stimulator cells, pulsed for 60 minutes with the H-2K$^d$-restricted peptide epitope NP147-155 (Thr-Tyr-Gln-Arg-Thr-Arg-Ala-Leu-Val, SEQ ID NO: 13) at 10 µM or infected with influenza strain A/Victoria/73, and 10 U/ml recombinant human IL-2 (Cellular Products, Buffalo, NY) were added and cultures were maintained for 7 days at 37°C with 5% CO2 and 100% relative humidity. The cytotoxicity assays were performed as described by Ulmer et al. (1993, *Science* 259:1745-1749). Target cells labeled with Na$^{51}$CrO were pulsed with synthetic peptide NP147-155 at a concentration of 10 µM. The target cells were then mixed with CTL at designated effector:targer cell ratios in 96-well plates, and incubated at 37°C for four hours in the presence of 5% CO2. A 20 µl sample of supernatant from each cell mixture was counted to determine the amount of $^{51}$Cr released from target cells and counted in a Betaplate scintillation counter (LKB-Wallac, Turku, Finland). Maximal counts, released by addition of 6M HCl, and spontaneous counts released without CTL were determined for each target preparation. Percent specific lysis was calculated as: [(E -S)/(M -S] x 100, where E represents the average cpm released from target cells in the presence of effector cells, S is the spontaneous cpm released in the presence of media only, and M is the maximum cpm released in the presence of 2% Triton X-100. The results in Figure 6A, Figure 6B, Figure 6C and Figure 6D show a minimal effect of the presence of AlPO4 on induction of an CTL response by innoculation with a NP DNA plasmid construct. In these studies BALB/c mice were injected in the quadriceps of both legs with plasmid DNA encoding A/ PR/8/34 (H1N1) with either 5 µg or 50µg of plasmid DNA, in PBS or AlPO4. The level of % specific lysis was determined through lymphocyte cultures derived from mice 6 weeks post injection. The results show that the CTL response was similar at both doses for both peptide-pulsed cells and flu-infected cells. Similar results were obtained for 5 µg or 50 µg doses at 3 weeks post 2 injections.

EXAMPLE 7

EFFECT OF ALUMINUM PHOSPHATE ON POTENCY OF HEPATITIS B DNA VACCINES

*DNA Constructions* - The major envelope protein (HBs) from hepatitis B virus was subcloned into expression vectors derived from V1J, derived from a pUC19 plasmid containing the human cytomegalovirus (CMV) immediate early promoter with its intron A sequence, multiple restriction sites (including Bgl II) for cloning and the bovine growth hormone polyadenylation signal sequence. The HB DNA plasmid vector expressing the *adw* subtype is V1Jns.S. The HB DNA plasmid vector expressing the *ayw* subtype is V1R.S, which was prepared by subcloning the S gene from a pBR322 plasmid that contained the entire HBV genome into the BglII restriction site of the V1R. Expression of the S gene was confirmed in RD cells (a human myoblast cell line) by calcium phosphate-mediated transfection using the CellPhect kit (Pharmacia) and detection of the HBsAg using the Auzyme EIA kit (Abbott Labs).

*Anti-HBs EIA (total antibody)* - A microtiter plate modification of the AUSAB EIA kit (Abbott Labs, N. Chicago, IL) was used to quantify antibodies to hepatitis B surface antigen (HBsAg). Costar EIA 96-well flat bottom plates (Costar, Cambridge MA, #3591) were coated overnight at 4°C with recombinant HBsAg (prepared *e.g.*, U.S.Patent Nos. 4,769,238; 4,935,235; and 5,196,194) at 4 µg/ml in Tris-saline, pH 9.5. Plates were washed 3 times with PBS and then blocked with 175 µl/well of PBS/5% FCS/ 0.1% azide for 2 hours at room temperature or overnight at 4°C. Five-fold serial dilutions were made (in duplicate) in 8 consecutive wells of the plate for each serum sample. The plates were then incubated overnight at 4°C. After 3 wash cycles with PBS (using a TiterTech plate washer [ICN, Huntsville, AL]), a developing reagent (Abbott AUSAB EIA kit) consisting of equal volumes of biotin-conjugated HBsAg and an anti-biotin-enzyme conjugate was added to each well of the plate. After 4 hours at room temperature, the plates were washed 6 times and then 100 µl per well of OPD substrate (Abbott) was added to each well. The reaction was stopped after 30 minutes with the addition of 50 µl per well of 1 N $H_2SO_4$. Optical densities were read at 490 nm and 650 nm using a Molecular Devices microplate reader (Molecular Devices, Menlo Park, CA). Anti-HBs titers (in mIU/mL) were calculated by the Softmax computer program (version 2.32) using a standard curve generated using a 4-parameter fit algorithm. Since the assay is species-independent, a set of human serum standards (Abbott quantitation kit) was used to generate the standard curve so that titers could be quantified relative to a reference standard in mIU/mL.

*Anti-HBs EIA (isotype-specific)* - Microtiter plates were coated with HBsAg and blocked as described above. Five-fold serial dilutions were made (in duplicate) in 8 consecutive wells of the plate for each serum sample. The plates were then incubated overnight at 4°C. After 3 wash cycles with PBS (using a TiterTech plate washer), alkaline phosphatase-conjugated goat anti-mouse immunoglobulin reagents specific for mouse IgG1 or mouse IgG2a isotypes (Southern Biotechnology Associates, Birmingham, AL) were added at a final dilution of 1:2000. After 2 hours at 37°C, the plates were washed 6 times using a TiterTech plate washer, and then 60 µl per well of the enzyme substrate (p-nitrophenylphosphate [Sigma Chemical Co., St. Louis, MO] dissolved at 1 mg/mL in Tris saline, pH 9.5) was added. After 30 minutes at room temperature, the reaction was stopped with the addition of 60 µl/well of 3N NaOH. Optical densities were read at 405 nm using a Molecular Devices microplate reader. Data were collected using the Softmax computer program. A standard curve was generated using mouse monoclonal anti-HBs antibodies of the IgG1 (catalogue # 16021, Pharmingen, San Diego, CA) or IgG2a (cat. # 16011D, Pharmingen) isotypes.

Antibody concentrations relative to each isotype standard were calculated as described previously (Caulfield and Shaffer, 1984, *J. Immunol. Methods* 74: 205-215). Briefly, to calculate titers, an OD value of 0.1 units was set as the endpoint. The log 5 titer (t) is determined by interpolation using the following formula:

$$t=x-((0.1-L)/(H-L)$$

where L = OD value of the first log 5 dilution giving an OD value below 0.1; H = OD value of the log 5 dilution closest to, but above the cutoff (0.1); x = the well number that has the OD value L.

The antibody concentration (c) in experimental samples is determined by comparing the endpoint titer in experimental wells with that of the standard curve by the following formula:

$$c = A \times 5^{(t-s)}$$

where A = the antibody concentration of the standard; s = the log 5 titer of the standard; t = the log 5 titer of the unknown. For example, if the log 5 endpoint titer of the standard (100 ng/ml) is 2.6 and the value of the unknown is 3.4, the concentration of antibody in the unknown would be:

$$c = 100 \times 5^{(3.4-2.6)} = 362 \text{ ng/ml}.$$

*Cytotoxic T Lymphocyte Assays (CTL assays)* - The CTL assays were performed as reported in Ulmer et al. (1993, *Science* 259: 1745-1749), and essentially as described in Example Section 6. Briefly, BALB/c mice were injected twice with a vaccine formulation consisting of HBV DNA plus aluminum phosphate or with naked HBV DNA. A single cell suspension of effector cells was then prepared and cultured *in vitro* with HBs peptide (28-39)-pulsed syngeneic stimulator cells. The cell suspension was assayed 7 days later for CTL activity against $^{51}$Cr-labeled P815 cells.

The syngeneic stimulator cells were prepared as a single cell suspension from the spleens of unimmunized BALB/c mice as follows. After lysis of red blood cells with ammonium chloride buffer (Gibco BRL ACK buffer), the cells were washed by centrifugation for 10 minutes at 1200 rpm (Jouan centrifuge model CR422), resuspended in DMEM culture medium (Gibco BRL #11965-092), and then irradiated using a $^{60}$Co source to deliver 2,000 - 4,000 rads. The cells were then pulsed with a 10 µM final concentration of the H-2 $K^d$ peptide HBs (28-39) (Chiron Mimetopes, Clayton, Victoria, Australia) which has the sequence Ile-Pro-Gln-Ser-Leu-Asp-Ser-Trp-Trp-Try-Ser-Leu [SEQ ID NO:14] (Schirmbeck et al., 1994, *J. Virol.* 68: 1418-1425). The cells were mixed approximately every 20 minutes for 1.5 - 2.5 hours and then washed 3 times with RPMI-1640 medium. Effector cells were prepared as single cell suspensions from spleens of immunized mice as described and then co-cultured with an approximately equal number of peptide-pulsed stimulator cells for 7 days at 37° C (5% $CO_2$) in "K" medium.

P815 (H-2[d]) mouse mastocytoma cells (ATCC, Rockville, MD) were radiolabeled by overnight culture with 0.5 - 1.2 mCi $^{51}$Cr (Amersham, cat. # CJS.4) added to 75 cm$^2$ culture flasks (Costar #3376) containing $\sim$ 5 x 10$^5$ cells per mL in a volume of 10 mL. The labeled cells were centrifuged at 1200 rpm for 5 minutes and the supernatant removed by aspiration. The cells were washed, counted, resuspended in DMEM culture medium at $\sim$ 10$^6$ cells per mL and then pulsed with 10 µM HBs (28-39) peptide at 37° C for 2-3 hr with frequent mixing. The target cells were then washed and adjusted to 10$^5$ cells per mL for plating. Meanwhile, effector cells from the 7 day restimulation cultures were harvested, washed, and added to triplicate wells of V bottom microtiter plates (Costar #3898) at 60 x 10$^5$, 30 x 10$^5$, 15 x 10$^5$, and 7.5 x 10$^5$ cells per mL. The $^{51}$Cr-labeled target cells were plated at 10$^4$ cells per well in 100 µl "K" medium to achieve effector:target ratios of 60:1, 30:1, 15:1, and 7.5:1. Triplicate wells containing only target cells cultured in 0.2 mL of medium served as controls for spontaneous $^{51}$Cr release whereas triplicate wells containing target cells cultured in 0.2 mL of medium containing 1.0 % Triton X-100 detergent (Sigma #T6878) served as controls for maximum $^{51}$Cr release. The plates were incubated for 4 hours at 37°C in a 5% $CO_2$ incubator and then centrifuged at 1200 rpm for 5 minutes to pellet the remaining target cells. The supernatants (20 µl) were then harvested using an Impact multichannel pipetor (Matrix Technology, Lowell MA, model #6622) and then transferred to Betaplate filter mats (Wallac #1205-402). The mats were dried and then transferred to plastic bags which were sealed after the addition of $\sim$11 mL of scintillation fluid. A Betaplate model 1205 scintillation counter (Wallac) was used to quantify the radioactive $^{51}$Cr contained in each spot on the mat corresponding to each well of the original 96-well plate. The % specific lysis was determined as set forth in Example Section 7.

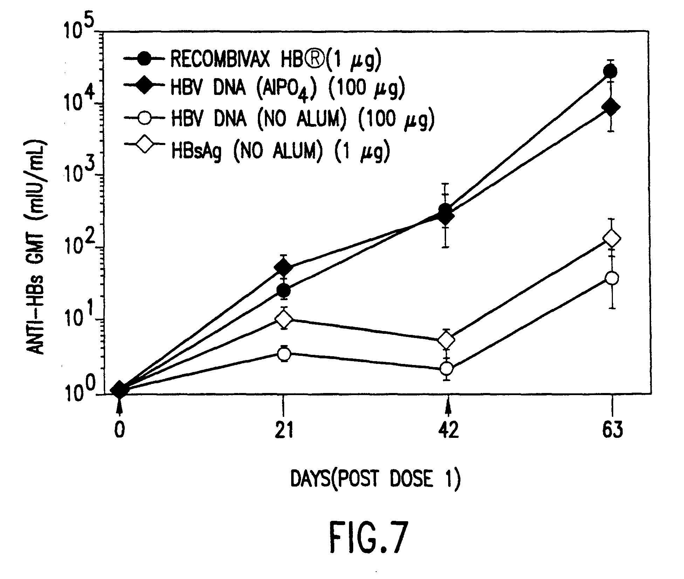

*Adjuvant effect of aluminum phosphate for V1R.S* - A study comparing anti-HBs antibody production in mice inoculated with (1) a commercial hepatitis B vaccine (Recombivax HB®); (2) purified hepatitis B surface antigen without an adjuvant, and (3) V1R.S with aluminum phosphate and (4) V1R.S without aluminum phosphate was performed. Animals were utilized as described in Example Section 6. Female BALB/c mice were inoculated with the plasmid DNA construct V1R.S at a 100 µg dose either in the presence of 450 µg /ml aluminum phosphate or in the absence of the adjuvant. As controls, one microgram of Recombivax HB® and 1 µg of HBsAg were injected into mice and bleeds were taken 21, 42 and 63 days after inoculation. Anti-HBs antibody production is shown in Figure 7. The antibody response to a HBV DNA vaccine (which encodes the surface antigen from hepatitis B virus) was enhanced approximately 100-fold by formulation with aluminum phosphate. The adjuvanted DNA vaccine generates a response equivalent to that induced with Recombivax HB® .

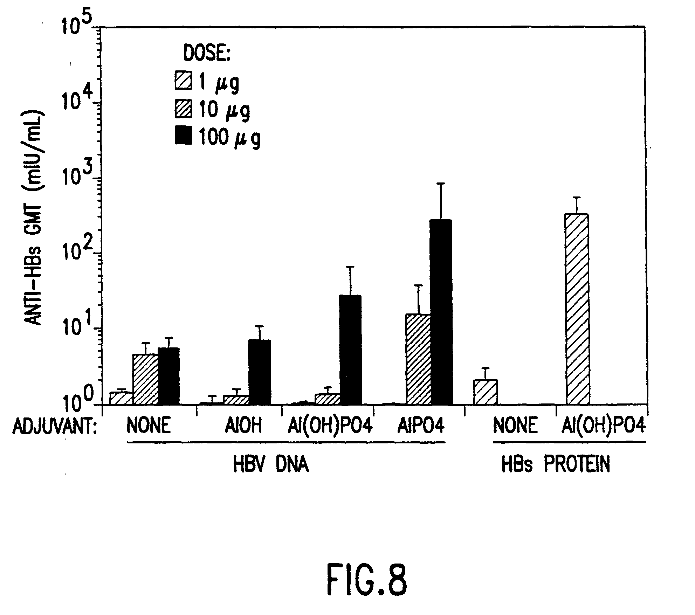

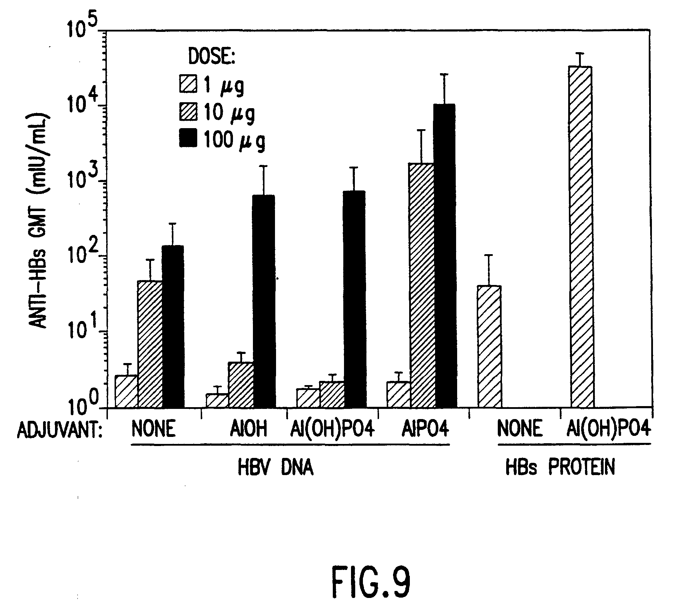

*HB DNA Doseage Rates in the Presence of AlPO4* - V1R.S DNA was formulated at three dose levels (1.0, 10, and 100 µg) with a constant (450 µg/ml) concentration of aluminum adjuvants (aluminum phosphate and aluminum hydroxyphosphate) and then tested for the ability to induce anti-HBs antibodies in mice. Figure 8 shows that 6 weeks after a single injection of vaccine, the response to a 10 µg dose of HBV DNA vaccine formulated with aluminum phosphate was superior to that induced with 100 µg of the naked DNA vaccine. Figure 9 shows that injection of mice at day 0 and day 42 with DNA formulated at three dose levels (1.0, 10, and 100 µg) with a constant (450 µg/mL) concentration of aluminum adjuvants. Anti-HBs antibodies in BALB/c mice were tested three weeks later at day 63 of the experiment. By comparison with the data shown in Figure 7, boosting with a second dose of DNA vaccine formulated with aluminum phosphate generated a > 10-fold rise in anti-HBs titers. Consistent with a single dosing as shown in Figure 7, the response to a 10 µg dose of HBV DNA vaccine formulated with aluminum phosphate was superior to that induced with 100 µg of the naked DNA vaccine. Formulation of DNA in saline with aluminum hydroxide or aluminum hydroxyphosphate adjuvants was advantageous only at the 100 µg dose of DNA under conditions in which the aluminum adjuvants are saturated and free DNA is present. At lower doses of DNA where it is known that the DNA binds completely to aluminum hydroxide or aluminum hydroxyphosphate, the response is lower than that obtained with equivalent doses of naked DNA.

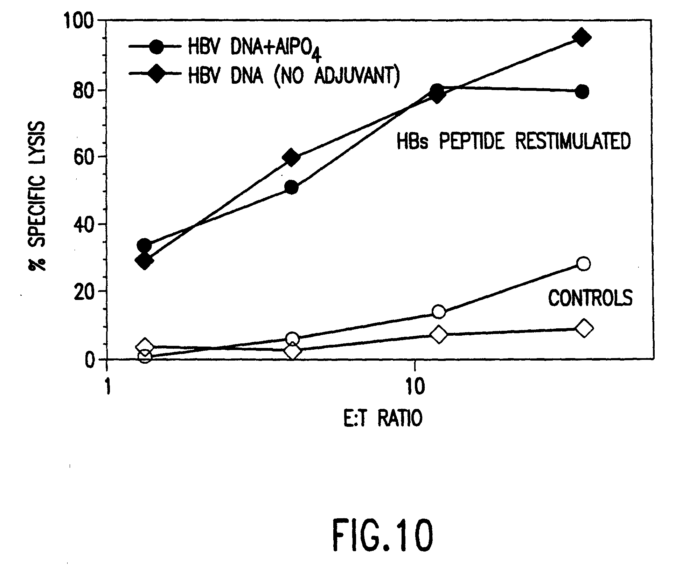

*HBV DNA /AlPO4 Induction of CTL Response* - After two injections of the HBV DNA vaccine plus aluminum phosphate adjuvant, spleen cells from BALB/c mice were restimulated *in vitro* with HBs peptide (28-39) and then assayed 7 days later for CTL activity against $^{51}$Cr-labeled P815 cells. Figure 10 shows that the formulation of the HBV DNA vaccine with or without aluminum phosphate generated equivalent CTL responses. There was no lysis of control P815 cells not pulsed with the HBs peptide indicating that lysis of the HBs peptide-pulsed cells was the result of activation of specific CTLs rather than natural killer (NK) cells that would be expected to lyse target cells indiscriminately. Therefore, a major advantages of naked DNA vaccination (i.e., induction of CTL responses) is preserved when the DNA is formulated with aluminum phosphate.

EXAMPLE 8

ADJUVANT EFFECT OF ALUMINUM PHOSPHATE FOR HBs DNA VACCINE TESTED IN LOW RESPONDER MICE

A significant proportion of humans are non-responders to a standard 3-dose regimen of the current hepatitis B vaccines (Alper, et al., 1989, *J. Eng. J. Med.* 321:708-712). This problem was addressed using the aluminum phosphate adjuvant in a preclinical animal model. Low responder (C3H) or high responder (BALB/c) mice were immunized with two doses of 1.0, 10, or 100 μg of HBs DNA vaccine formulated with or without aluminum phosphate. As shown in Table 9, formulation of the DNA vaccine with aluminum phosphate enables the generation of an anti-HBs antibody response in both high responder (BALB/c) and low responder (C3H) mice given the 100 μg dose of DNA that is equivalent to the response to a 1 μg dose of a conventional HBs protein vaccine. It is of note that in the absence of the aluminum adjuvant, the response to the DNA vaccine was only 6.3 mIU/mL which is just above the detectable limit of ~1.0 mIU per mL. Thus, the aluminum phosphate adjuvant combines the desired attributes of protein-based vaccines (i.e. the induction of high antibody titers) with the ability of DNA vaccines to induce cell-mediated antibody responses (see Example Section 7).

TABLE 9

| Anti-HBs response of high vs. low responder mice to HBsDNA ± AIPO4 | | | | |
|---|---|---|---|---|
| Immunogen | Dose | Adjuvant | Anti-HBs GMT (mIU/mL) | |
| | | | BALB/c | C3H |
| HBsAg | 1 μg | Al(OH)PO4 | 8,045.0 | 91.4 |
| HBs DNA* | 100μg | none | 415.0 | 6.3 |
| | 10μg | | 19.8 | 1.7 |
| | 1μg | | 3.3 | 1.3 |
| | 100μg | AIPO4 | 4,408.0 | 111.5 |
| | 10μg | | 280.0 | 23.7 |
| | 1μg | | 6.8 | 4.8 |

*V1R.S (ayw) (XLpl.11) 5286-115 V44

EXAMPLE 9

EFFECT OF ALUMINUM PHOSPHATE ON POTENCY OF HIV DNA VACCINES

DNA plasmid V1Jns/tPA/opt gag was constructed from the vector V1Jns, described in WO 97/3115 and herein incorporated by reference. The optimized gag sequence within V1Jns was constructed as follows: In order to provide an heterologous leader peptide sequence to secreted and/or membrane proteins, V1Jn was modified to include the human tissue-specific plasminogen activator (tPA) leader. Two synthetic complementary oligomers were annealed and then ligated into V1Jn which had been BglII digested. These oligomers have overhanging bases compatible for ligation to BglII-cleaved sequences. After ligation the upstream BglII site is destroyed while the downstream BglII is retained for subsequent ligations. Both the junction sites as well as the entire tPA leader sequence were verified by DNA sequencing. Additionally, in order to conform with our consensus optimized vector V1Jns (=V1Jneo with an SfiI site), an SfiI restriction site was placed at the KpnI site within the BGH terminator region of V1Jn-tPA by blunting the KpnI site with T4 DNA polymerase followed by ligation with an SfiI linker (catalogue #1138, New England Biolabs). This modification was verified by restriction digestion and agarose gel electrophoresis.

Gene segments were converted to sequences having identical translated sequences but with alternative codon usage as defined by Lathe (1985, *J. Mol. Biol.* 183: 1-12), and described in WO 97/3115. The methodology described below to increase of expression of HIV *gag* gene segments was based on the hypothesis that the known inability to express this gene efficiently in mammalian cells is a consequence of the overall transcript composition. Thus, using alternative codons encoding the same protein sequence may remove the constraints on expression of *gag*. The specific codon replacement method employed may be described as follows:

    1. Identify placement of codons for proper open reading frame.

2. Compare wild type codon for observed frequency of use by human genes.

3. If codon is not the most commonly employed, replace it with an optimal codon for high expression in human cells.

4. Repeat this procedure until the entire gene segment has been replaced.

5. Inspect new gene sequence for undesired sequences generated by these codon replacements (e.g., "ATTTA" sequences, inadvertent creation of intron splice recognition sites, unwanted restriction enzyme sites, etc.) and substitute codons that eliminate these sequences.

6. Assemble synthetic gene segments and test for improved expression.

These methods were used to create the following synthetic gene segments for HIV *gag* creating a gene comprised entirely of optimal codon usage for expression. An artisan of ordinary skill in the art will understand that similar vaccine efficacy or increased expression of genes may be achieved by minor variations is the procedure or by minor variations in the sequence.

DNA plasmid V1Jns/tPA/gp140 optA was constructed as described above for optimization and specifically as described in PCT International Application No. WO 97/31115.

Female Balb/C mice (10/group) were inoculated with V1Jns/tPA/gp140optA and V1Jns/tPAopt gag at doses of 10 µg (5 µg of each construct) or 100 µg (50 µg of each construct). Aluminum phosphate (AlPO4 from a 2% solution), or CaPO4 (27.5mg/100ml stock) was added at final amounts of 11 µg for AlPO4, and 19 µg for CaPO4. Controls included inoculations formulations with adjuvant and/or no DNA or DNA with no adjuvant.

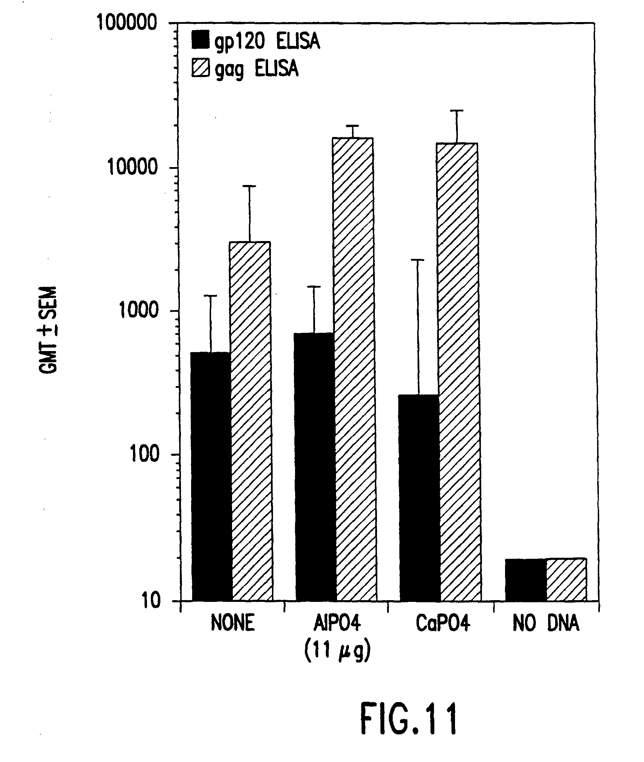

Figure 11 shows the effects of various adjuvants with a HIV eng/gag DNA vaccine formulation on gp120 and gag antibody responses in inoculated mice. Antibody production was measured by ELISA. As shown in Example 7 with HBV DNA vaccines, CTL responses with and without AlPO4 were approximately equal. Therefore, use of an adjuvanted HIV env/gag formulation did not decrease the ability of the vaccine to promote a specific CTL response.

EXAMPLE 10

DOSE-RESPONSE RELATIONSHIP OF CALCIUM PHOSPHATE AS AN ADJUVANT FOR A DNA VACCINE.

Calcium phosphate (at different concentrations) was compared with a standard concentration of aluminum phosphate as an adjuvant for HBV DNA vaccine. Three dose levels (10, 100, and 1000 µg/mL) of the HBV DNA vaccine were formulated to contain 10, 3.3, 1.0, or 0.3 mg/mL calcium phosphate or 0.45 mg/mL aluminum phosphate. A total of 0.1 mL of formulated vaccines was injected into two intramuscluar sites of BALB/c mice to achieve DNA vaccine dosages of 1.0, 10, or 100 µg. As shown in Table 10, HBV DNA formulated with the three lower concentrations of calcium phosphate increased the anti-HBs response to the 100 µg vaccine dose by approximately 10-fold. At the highest concentration of calcium phosphate, only 7% of the DNA remained unbound to the adjuvant, and the response to this formulation was increased by less than 2-fold. In the cohorts of mice receiving the 10 µg dose of HBV DNA, the aluminum phosphate had a powerful adjuvant effect, increasing the response ~40-fold compared with the group receiving 10 µg of naked DNA. By contrast, DNA formulated with calcium phosphate at 10, 3.3, or 1 mg per mL induced a response that was 10-fold lower than that to naked DNA. Only in the group receiving the DNA vaccine formulated with the lowest concentration of calcium phosphate (0.3 mg/mL) was an adjuvant effect observed for the 10 µg dose level of the DNA vaccine. An analysis of separately prepared vaccines (10 µg DNA dose) containing calcium phosphate indicated that the percent unbound DNA was 73%, 26%, 1%, and 0% in vaccines containing 0.3, 1.0, 3.3, and 10 mg/mL calcium phosphate, respectively. Taken together, these results indicate that calcium phosphate can be an effective adjuvant for a DNA vaccine only if the formulation contains a substantial amount of free DNA. If the DNA dose is limiting or if the calcium phosphate concentration is excessive, the antibody response to the DNA vaccine formulation may be inhibited.

TABLE 10

| Adjuvant Effect of $CaPO_4$ vs $AlPO_4$ for HBs DNA or protein vaccines | | | | | | |
|---|---|---|---|---|---|---|
| | | | Anti-HBs mIU /mL (Vaccine Dose) | | | |
| Adjuvant | Dose (mg/mL) | Vaccine | (1 µg) | (10 µg) | (100 µg) | % unbound DNA (@ 100 µg dose) |
| none | 0 | HBs DNA | 1.9 | 111.0 | 313.0 | n.a. |
| AlPO4 | 0.45 | | 2.6 | 4,303.0 | 16,375.0 | ~100 |
| CaPO4 | 10 | | 4.0 | 4.5 | 556.0 | 7 |
| | 3.3 | | 1.0 | 1.2 | 4,370.0 | 64 |

TABLE 10   (continued)

| Adjuvant Effect of CaPO$_4$ vs AlPO$_4$ for HBs DNA or protein vaccines | | | | | | |
|---|---|---|---|---|---|---|
| | | | Anti-HBs mIU /mL (Vaccine Dose) | | | |
| Adjuvant | Dose (mg/mL) | Vaccine | (1 µg) | (10 µg) | (100 µg) | % unbound DNA (@ 100 µg dose) |
| | 1 | | 1.0 | 7.2 | 1,782.0 | 92 |
| | 0.3 | | 1.4 | 363.0 | 2,091.0 | ~100 |
| | 3.3 | HBs protein | 87,636.0 | n.d. | n.d. | n.a. |
| Al(OH)PO4 | 0.45 | | 105,240.0 | n.d. | n.d. | n.a. |
| V1R.S Plasmid DNA n = 10 BALB/c mice per group Injection route: 0.05 mL in 2 intramuscular sites Injection schedule: d. 0, 42 Assay time: d. 84 | | | | | | |

EXAMPLE 11

HBV DNA VACCINE / ALUMINUM PHOSPHATE FORMULATION AS A PRIMING ANTIGEN

A study comparing anti-HBs antibody production in mice primed with two doses of a hepatitis B DNA vaccine (V1Jns.S2.S at 100 µg per dose), (1) with aluminum phosphate, (2) without aluminum phosphate, and (3) Recombivax HB®. These priming immunogens were followed by either a boosting with either a V1Jns.S2.S or Recombivax HB®. Table 11 shows pre-boost and post-boost HBs antibody titers. DNA/AlPO4 priming followed by boosting with Recombivax HB® results in approximately a 300-fold increase in HBs antibody when compared to DNA priming (without aluminum phosphate) prior to boosting with Recombivax HB®.

TABLE 11

| Effective priming with HBV DNA + AlPO$_4$ for a booster response to a conventional protein vaccine | | | | |
|---|---|---|---|---|
| | | | Anti-HBs GMT (mIU/mL) | |
| Priming Immunogen (2 doses) | Adjuvant | Booster | pre-boost | post-boost |
| DNA | none | DNA | 14 | 39 |
| | | Protein | | 112 |
| DNA | AlPO$_4$ | DNA | 279 | 307 |
| | | Protein | | 39,750 |
| Protein | Al(OH)PO$_4$ | Protein | 11,100 | 106,606 |
| DNA vaccine 100 µg per dose): V1Jns.S2.S Protein vaccine (1 µg): Recombivax HB® | | | | |

EXAMPLE 12

EFFECT OF ALUMINUM PHOSPHATE ON POTENCY OF A HERPES SIMPLEX DNA VACCINE IN GUINEA PIGS

Plasmids V1Jns:gD and V1Jns:ΔgB encoding HSV-2 glycoprotein D (gD) and the amino-terminal 707 amino acids of glucoprotein B (gB), respectively have been described in McClements et al. (1996, *Proc Natl Acad Sci U S A* 93: 11414-11420). The vaccines were prepared by diluting V1Jns:gD DNA and V1Jns:ΔgB DNA into either sterile PBS, or sterile PBS containing AdjuPhos® at a final aluminum concentration of 450 µg/mL. Vaccines were thoroughly mixed by gentle vortexing then stored at 4°C for 24 hours. Immediately prior to injection, the vaccine formulations were subjected to gentle vortexing. Female Duncan Hartley guinea pigs (Harlan Sprague Dawley; Indianapolis, IN) weighing between 450-550 grams at the time of the first immunization were injected with a total of 200 µL (100 µL per quadriceps muscle) containing 6 µg V1Jns:gD + 20 µg V1Jns:ΔgB, with or without 90 µg aluminum. Animals were boosted at five weeks. Sera obtained at weeks 4 and 8 were assayed at ten-fold dilutions, ranging from 1:30 to 1:30,000, using gD- and gB-specific ELISAs (McClements et al, 1996, *Proc Natl Acad Sci U S A* 93: 11414-11420). Endpoint titers were determined as described previously except that serum dilutions were considered positive if the $OD_{450}$ signal was > 0.05 above that of the preimmune sera at the same dilution (McClements et al, 1996, *Proc Natl Acad Sci U S A* 93: 11414-11420). These results are presented in Table 12.

## TABLE 12

ELISA GMT; linear values (range) and $\log_{10}$ values ± SEM; N=4

| group | 4 wk | | 8 wk | |
|---|---|---|---|---|
| | anti gD | anti gB | anti gD | anti gB |
| DNA in PBS | 17 (9-33) | 53 (10-190) | 53 (10-284) | 5335 (2744-10370) |
| | 1.23 ± .29 | 1.73 ± .55 | 1.73 ± .73 | 3.73 ± .29 |
| DNA + AlPO₄ | 30 (10-89) | 949 (251-3585) | 169 (17-1634) | 30000 (30K-30K)* |
| | 1.48 ± .49 | 2.98 ± .58 | 2.23 ± .99 | 4.48 ± 0 |

\* all strongly positive at highest dilution tested

EXAMPLE 13

EFFECT OF ALUMINUM PHOSPHATE ON POTENCY OF AN INFLUENZA DNA VACCINE IN PRIMATES

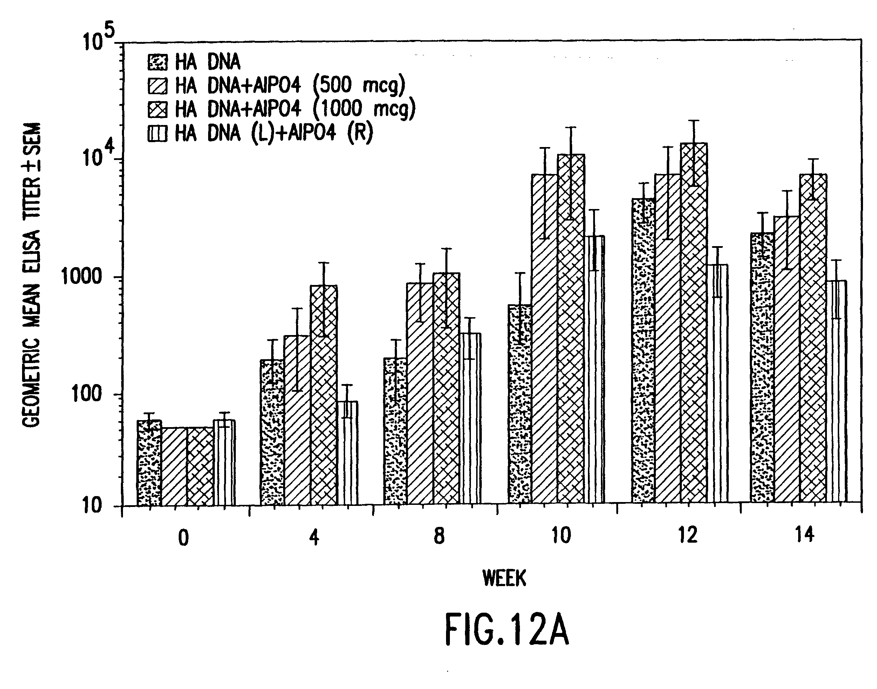

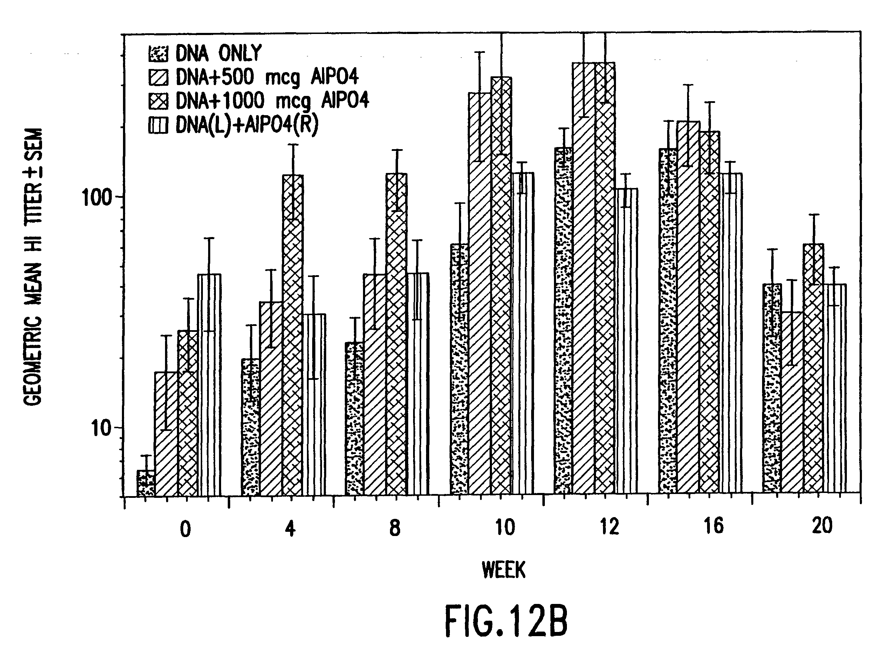

*Rhesus monkeys* - Groups of 5 young adult Rhesus of either sex were injected intramuscularly in both triceps muscles with 0.5 mL of a solution containing 500 mcg/mL of V1Jns-HA/Georgia plasmid encoding the HA from influenza A/Georgia/03/93 (H3N2), dissolved in phosphate-buffered saline or in phosphate-buffered saline with 500 mcg/mL or 1000 mcg/mL of aluminum phosphate adjuvant. A separate control group received HA DNA and aluminum in contralateral arms. Immunizations were given at 0 time and again at 8 weeks. Animals were bled at two week intervals and sera were tested for antibodies against A/Georgia/03/93 by hemagglutination inhibition (Figure 12A) and by ELISA (Figure 12B). Use of aluminum phosphate adjuvant in combination with the DNA increased antibody titers compared to animals that received DNA alone or DNA and adjuvant in contralateral arms. Repeated measures analysis of variance indicated that the pooled antibody titers of groups that received DNA mixed with aluminum were significantly higher than the pooled antibody titers of groups that received no aluminum or aluminum and DNA in contralateral arms ($P<0.05$).

Chimpanzee - Four adult chimpanzees of either sex were injected intramuscularly in one triceps muscle with a volume of 1.0 mL containing 500 mcg of V1Jns-HA/Georgia plasmid encoding the HA from influenza A/Georgia/03/93 (H3N2), dissolved in phosphate-buffered saline or in phosphate-buffered saline with 500 mcg of aluminum phosphate adjuvant. Immunizations were given at time 0 and at 6 and 12 weeks. Sera were collected at two week intervals and were assayed for antibodies by HI, virus neutralization, and ELISA. Table 13 shows that greater antibody responses were seen in the two animals given HA DNA with aluminum adjuvant, with 1/2 in the alum group having at least fourfold rises in HI antibody and 2/2 having fourfold rises in virus neutralization, while 0/2 animals given HA DNA alone exhibited these responses.

Table 13

Antibody Responses of Chimpanzees to HA DNA Vaccine ± Alum adjuvant

| Treatment | Animal | Week | Hi Titer vs. | | A/Georgia ELISA | A/Georgia Virus Neutralization |
| | | | A/Georgia | A/Guang-dong | | |
|---|---|---|---|---|---|---|
| HA DNA | X019 | 0 | 5 | 5 | 25 | 20 |
| | | 4 | 5 | 5 | 50 | 20 |
| | | 6 | 5 | 5 | 25 | 10 |
| | | 8 | 5 | 5 | 25 | 10 |
| | | 10 | 5 | 5 | 200 | 20 |
| | | 12 | 5 | 5 | 200 | 20 |
| | | 14 | 10 | 5 | 200 | 40 |
| HA DNA | X131 | 0 | 5 | 5 | 400 | 20 |
| | | 4 | 5 | 5 | 200 | 20 |
| | | 6 | 10 | 5 | 200 | 10 |
| | | 8 | 5 | 5 | 200 | 20 |
| | | 10 | 10 | 5 | 200 | 20 |
| | | 12 | 5 | 5 | 200 | 20 |
| | | 14 | 10 | 5 | 200 | 40 |
| HA DNA + AlPO4 | X133 | 0 | 5 | 5 | 50 | 10 |
| | | 4 | 5 | 5 | 50 | 20 |
| | | 6 | 10 | 5 | 100 | 20 |
| | | 8 | 20 | 40 | 1600 | 160 |
| | | 10 | 10 | 20 | 800 | 80 |
| | | 12 | 10 | 20 | 400 | 80 |
| | | 14 | 20 | 40 | 800 | 320 |
| HA DNA + AlPO4 | X140 | 0 | 5 | 5 | 100 | 10 |
| | | 4 | 5 | 5 | 100 | 5 |
| | | 6 | 5 | 5 | 200 | ND |
| | | 8 | 10 | 5 | 200 | 20 |
| | | 10 | 10 | 5 | 200 | 20 |
| | | 12 | 5 | 5 | 100 | 10 |
| | | 14 | 10 | 5 | 400 | 80 |

**Claims**

1.  A pharmaceutical formulation comprising:

    (a) a DNA polynucleotide vaccine encoding at least one antigen, such that introduction of said vaccine into a vertebrate host results in expression of a biologically effective amount of said antigen or antigens so as to induce a prophylactic or therapeutic immune response, and
    (b) an aluminium-based negatively charged adjuvant.

2.  A pharmaceutical formulation of claim 1 wherein the adjuvant is aluminium phosphate-based.

3.  A pharmaceutical formulation according to claim 2 wherein the nucleic acid molecules do not show substantial binding to the adjuvant.

4.  A pharmaceutical formulation according to claim 2 or claim 3 wherein the adjuvant possesses a molar $PO_4$/Al ratio of about 0.9.

5.  A pharmaceutical formulation according to any previous claim wherein the adjuvant is amorphous aluminium hydroxyphosphate gel.

6.  A pharmaceutical formulation according to any previous claim wherein said polynucleotide vaccine expresses said antigen or antigens so as to induce a prophylactic or therapeutic immune response against a disease or disorder selected from the group consisting of human immunodeficiency virus, herpes simplex virus, human influenza, hepatitis A, hepatitis B, hepatitis C, human papilloma virus, tuberculosis, tumor growth, autoimmune disorders and allergies.

7.  A pharmaceutical formulation according to any of claims 1-5 wherein said polynucleotide vaccine expresses said antigen or antigens so as to induce a prophylactic or therapeutic immune response against a veterinary disease or disorder selected from the group consisting of rabies, distemper, foot and mouth disease, anthrax, bovine herpes simplex and bovine tuberculosis.

8.  A pharmaceutical formulation according to any of claims 1-5 wherein said polynucleotide vaccine is a DNA plasmid.

9.  A pharmaceutical formulation according to any previous claim adapted for parenteral, inhalation, or oral delivery.

10. A pharmaceutical formulation according to claim 9 adapted for intramuscular administration.

11. The use of:

    (a) a polynucleotide vaccine encoding at least one antigen;
    and
    (b) an aluminium-based negatively charged adjuvant for the manufacture of a medicament for inducing a prophylactic or therapeutic immune response against a disease or disorder selected from the group consisting of human immunodeficiency virus, herpes simplex virus, human influenza, hepatitis A, hepatitis B, hepatitis C, human papilloma virus, tuberculosis, tumor growth, autoimmune disorders and allergies.

12. The use of:

    (a) a polynucleotide vaccine encoding at least one antigen;
    and
    (b) an aluminium-based negatively charged adjuvant for the manufacture of a medicament for inducing a prophylactic or therapeutic immune response against a disease or disorder selected from the group consisting of rabies, distemper, foot and mouth disease, anthrax, bovine herpes simplex and bovine tuberculosis.

**Patentansprüche**

1.  Pharmazeutische Formulierung, umfassend:

a) ein DNA-Polynukleotid-Vakzin, das für mindestens ein Antigen kodiert, so daß die Einführung des Vakzins in einen Vertebratenwirt zur Expression einer biologisch wirksamen Menge des Antigens oder der Antigene führt, um eine prophylaktische oder therapeutische Immunreaktion zu induzieren, und
b) ein negativ geladenes Adjuvans auf Aluminiumbasis.

2. Pharmazeutische Formulierung nach Anspruch 1, wobei das Adjuvans auf Aluminiumphosphat basiert.

3. Pharmazeutische Formulierung nach Anspruch 2, wobei die Nukleinsäuremoleküle keine substantielle Bindung an das Adjuvans zeigen.

4. Pharmazeutische Formulierung nach Anspruch 2 oder Anspruch 3, wobei das Adjuvans ein molares $PO_4$/Al-Verhältnis von etwa 0,9 aufweist.

5. Pharmazeutische Formulierung nach irgendeinem der vorhergehenden Ansprüche, wobei das Adjuvans ein amorphes Aluminiumhydroxyphosphat-Gel ist.

6. Pharmazeutische Formulierung nach irgendeinem der vorhergehenden Ansprüche, wobei das Polynukleotid-Vakzin das Antigen oder die Antigene exprimiert, um eine prophylaktische oder therapeutische Immunreaktion gegen eine Krankheit oder Störung zu induzieren, welche aus der Gruppe bestehend aus Human-Immunschwäche-Virus, Herpes-Simplex-Virus, Human-Influenza, Hepatitis A, Hepatitis B, Hepatitis C, Human-Papilloma-Virus, Tuberkulose, Tumorwachstum, Autoimmunerkrankungen und Allergien ausgewählt ist.

7. Pharmazeutische Formulierung nach irgendeinem der Ansprüche 1-5, wobei das Polynukleotid-Vakzin das Antigen oder die Antigene exprimiert, um eine prophylaktische oder therapeutische Immunreaktion gegen eine veterinärmedizinische Krankheit oder Störung zu induzieren, welche aus der Gruppe bestehend aus Rabies (Tollwut), Staupe, Maul- und Klauenseuche, Milzbrand, Rinder-Herpes-Simplex und Rinder-Tuberkulose ausgewählt ist.

8. Pharmazeutische Formulierung nach irgendeinem der Ansprüche 1-5, wobei das Polynukleotid-Vakzin ein DNA-Plasmid ist.

9. Pharmazeutische Formulierung nach irgendeinem der vorhergehenden Ansprüche, welche für die parenterale oder orale Abgabe oder Abgabe mittels Inhalation adaptiert ist.

10. Pharmazeutische Formulierung nach Anspruch 9, adaptiert für die intramuskuläre Verabreichung.

11. Verwendung von:

a) einem Polynukleotid-Vakzin, kodierend für mindestens ein Antigen, und
b) einem negativ geladenen Adjuvans auf Aluminiumbasis zur Herstellung eines Medikaments zur Induktion einer prophylaktischen oder therapeutischen Immunreaktion gegen eine Erkrankung oder Störung, die aus der Gruppe, bestehend aus Human-Immunschwäche-Virus, Herpes-Simplex-Virus, Human-Influenza, Hepatitis A, Hepatitis B, Hepatitis C, Human-Papilloma-Virus, Tuberkulose, Tumorwachstum, Autoimmunerkrankungen und Allergien ausgewählt ist.

12. Verwendung von:

a) einem Polynukleotid-Vakzin, kodierend für mindestens ein Antigen, und
b) einem negativ geladenen Adjuvans auf Aluminiumbasis zur Herstellung eines Medikaments zur Induktion einer prophylaktischen oder therapeutischen Immunreaktion gegen eine Erkrankung oder Störung, die aus der Gruppe, bestehend aus Rabies (Tollwut), Staupe, Maul- und Klauenseuche, Milzbrand, Rinder-Herpes-Simplex und Rinder-Tuberkulose ausgewählt ist.

**Revendications**

1. Formulation pharmaceutique comprenant:

(a) un vaccin à base de polynucléotide d'ADN codant' pour au moins un antigène, tel que l'introduction dudit vaccin dans un hôte vertébré se traduit par l'expression d'une quantité biologiquèment efficace du ou desdits antigènes afin d'induire une réponse immunitaire prophylactique ou thérapeutique, et

(b) un adjuvant chargé négativement à base d'aluminium.

2. Formulation pharmaceutique selon la revendication 1, dans laquelle l'adjuvant est à base de phosphate d'aluminium.

3. Formulation pharmaceutique selon la revendication 2, dans laquelle les molécules d'acide nucléique ne présentent pas de liaison substantielle avec l'adjuvant.

4. Formulation pharmaceutique selon la revendication 2 ou la revendication 3, dans laquelle l'adjuvant possède un rapport molaire $PO_4$/Al d'environ 0,9.

5. Formulation pharmaceutique selon l'une quelconque des revendications précédentes, dans laquelle l'adjuvant est un gel d'hydroxyphosphate d'aluminium amorphe.

6. Formulation pharmaceutique selon l'une quelconque des revendications précédentes, dans laquelle ledit vaccin polynucléotidique exprime le ou lesdits antigènes afin d'induire une réponse immunitaire prophylactique ou thérapeutique contre une maladie ou un trouble choisi dans le groupe constitué par le virus de l'immunodéficience humaine, l'herpèsvirus simplex, la grippe humaine, l'hépatite A, l'hépatite B, l'hépatite C, le papillomavirus humain, la tuberculose, la croissance tumorale, les maladies auto-immunes et les allergies.

7. Formulation pharmaceutique selon l'une quelconque des revendications 1 à 5, dans laquelle ledit vaccin polynucléotidique exprime le ou lesdits antigènes afin d'induire une réponse immunitaire prophylactique ou thérapeutique contre une maladie ou un trouble vétérinaire choisi dans le groupe constitué par la rage, la maladie de Carré, la fièvre aphteuse, l'anthrax, l'herpès virus simplex bovin et la tuberculose bovine.

8. Formulation pharmaceutique selon l'une quelconque des revendications 1 à 5, dans laquelle ledit vaccin polynucléotidique est un plasmide d'ADN.

9. Formulation pharmaceutique selon l'une quelconque des revendications précédentes, adaptée pour la délivrance par voie parentérale, d'inhalation ou orale.

10. Formulation pharmaceutique selon la revendication 9, adaptée pour l'administration intramusculaire.

11. Utilisation de :

(a) un vaccin polynucléotidique codant pour au moins un antigène ; et
(b) un adjuvant chargé négativement à base d'aluminium pour fabriquer un médicament destiné à induire une réponse immunitaire prophylactique ou thérapeutique contre une maladie ou un trouble choisi dans le groupe constitué par le virus de l'immunodéficience humaine, herpèsvirus simplex, la grippe humaine, l'hépatite A, l'hépatite B, l'hépatite C, le papillomavirus humain, la tuberculose, la croissance tumorale, les maladies auto-immunes et les allergies.

12. Utilisation de :

(a) un vaccin polynucléotidique codant pour au moins un antigène ; et
(b) un adjuvant chargé négativement à base d'aluminium pour fabriquer un médicament destiné à induire une réponse immunitaire prophylactique ou thérapeutique contre une maladie ou un trouble choisi dans le groupe constitué par la rage, la maladie de Carré, la fièvre aphteuse, l'anthrax, l'herpèsvirus simplex bovin et la tuberculose bovine.

FIG.1A

FIG.1B

FIG.2A

FIG.2B

FIG.3A

FIG.3B

FIG.4

EP 1 017 283 B1

FIG.5A

FIG.5B

FIG.5C

FIG.5D

FIG.6A

FIG.6B

FIG.6C

FIG.6D

FIG.7

FIG.8

FIG.9

FIG.10

FIG.11

FIG.12A

EP 1 017 283 B1

FIG.12B